# EUROPEAN PATENT APPLICATION

(11) **EP 4 566 449 A1**
(43) Date of publication of application: **11.06.2025**
(21) Application number: 23849471.0
(22) Date of filing: 02.08.2023
(51) Int. Cl.: A01N 43/54, A01N 37/00, C07D 239/54, C07D 413/04, A01P 13/00

(54) **HERBICIDE COMPOSITION COMPRISING URACIL COMPOUND COMPRISING CARBOXYLATE FRAGMENT AND USE OF HERBICIDE COMPOSITION**

(30) Priority: 04.08.2022 CN 202210935789
(71) Applicant: Jiangsu Flag Chemical Industry Co., Ltd, Nanjing, Jiangsu 210000 (CN)
(72) Inventor: ZHANG, Pu, Nanjing, Jiangsu 210000 (CN); XU, Dan, Nanjing, Jiangsu 210000 (CN); YAO, Kaicheng, Nanjing, Jiangsu 210000 (CN); BAI, Congqiang, Nanjing, Jiangsu 210000 (CN); WU, Yaojun, Nanjing, Jiangsu 210000 (CN)
(74) Representative: Sun, Yanan
(86) International application number: PCT/CN2023/110773
(87) International publication number: WO 2024/027767

(57) **Abstract**

The present invention relates to a herbicide composition containing at least one of a uracil compound containing a carboxylate fragment of formula (I) or an agricultural salt thereof or a derivative thereof and at least one compound with herbicidal activity and the use thereof, wherein each variable is defined in the specification.

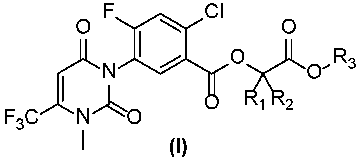

## Description

### TECHNICAL FIELD

The present invention relates to the field of herbicides, in particular to a herbicide composition of a uracil compound containing a carboxylate fragment and the use thereof.

### BACKGROUND

Chemical control of weed is a most economical and effective means for controlling weeds in farmlands, however, continuously use of a single chemical herbicide or chemical herbicides having a single functional mechanism at a high dosage for a long period of time is likely to cause problems associated with evolved drug resistance and tolerance of weeds. Rational compounding or mixing the herbicide compounds can achieve the following advantages: exhibiting a broad spectrum of weed control, improving weed control effect, and delaying occurrence and development of drug resistance and tolerance of weeds, and thus is one of the most effective means to solve the above problems. Some uracil compounds containing a carboxylate fragment of the following formula with a herbicidal activity are already known from CN114621150A, however, a species of herbicide composition having a high safety, a broad weeding spectrum, being able to generate a synergistic effect, as well as solving the problem of resistant weeds still needs to be developed in production.

Special combinations of the herbicides having different action mechanisms are able to increase the activity of the herbicide composition through the synergistic effect. In such a way, the amount of the herbicide required to control the weeds can be reduced.

Some uracil compounds containing a carboxylate fragment of the following formula with a herbicidal activity are already known from CN114621150A. However, there is no report on the composition of such compounds and other herbicides.

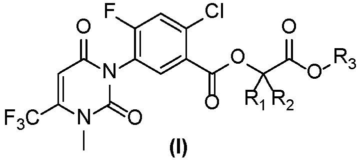

### SUMMARY

The objective of the present invention is to provide a herbicide composition of a uracil compound containing a carboxylate fragment and the use thereof.

The technical solution of the present invention to solve the above technical problems is as follows:
A herbicide composition of a uracil compound containing a carboxylate fragment, comprising an active ingredient A and an active ingredient B; wherein the active ingredient A is at least one compound indicated in formula (I), and the active ingredient B is at least one of a compound with a herbicidal activity or a salt thereof or a derivative thereof, different from the compound indicated in formula (I); a weight ratio of the active ingredient A to the active ingredient B is from 1:500 to 500:1; a structure of the active ingredient A is as follows: 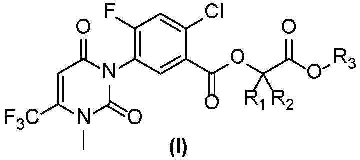 wherein,
R₁ and R₂ are each independently selected from hydrogen or methyl, preferably selected from methyl; R₃ is selected from C_{1∼6} alkyl, C_{1∼6} halogen-substituted C_{1∼6} alkyl, C_{3~6} alkenyl, C_{3~6} alkenyl substituted by one or more halogens, C_{3~6} alkynyl, C_{3~6} alkynyl substituted by one or more halogens, C_{3~6} cycloalkyl, C_{3~6} cycloalkyl C_{1~3} alkyl, C_{1~3} alkoxy C_{1~3} alkyl, C_{1~3} haloalkoxy C_{1~3} alkyl, C_{2~6} alkenoxy C_{1~3} alkyl, C_{2~6} haloalkenoxy C_{1~3} alkyl, C_{2~6} alkynoxy C_{1~3} alkyl, C_{2~6} haloalkynoxy C_{1~3} alkyl , C_{1~3} alkyl S(O)ₙ C_{1~3} alkyl, C_{3~6} oxygen containing cycloalkyl or C_{3~6} oxygen containing cycloalkyl C_{1~3} alkyl, n=0, 1 or 2, preferably selected from C_{1∼6} alkyl, C_{3~6} alkenyl, C_{3~6} alkynyl, C_{3~6} cycloalkyl, C_{3~6} cycloalkyl C_{1~3} alkyl, C_{1~3} alkoxy C_{1~3} alkyl, C_{2~6} alkenoxy C_{1~3} alkyl, C_{2~6} alkynoxy C_{1~3} alkyl, C_{1~3} alkyl S(O)ₙ C_{1~3} alkyl, C_{3~6} oxygen containing cycloalkyl or C_{3~6} oxygen containing cycloalkyl C_{1~3} alkyl, n=0, 1 or 2, more preferably selected from C_{1∼6} alkyl, C_{1~3} alkoxy C_{1~3} alkyl, C_{2~6} alkenoxy C_{1~3} alkyl; when R₁ is selected from hydrogen and R₂ is selected from methyl, the chiral carbon atom connected thereto is selected from either an R configuration or an S configuration, or a mixture of the two;
the active ingredient B is selected from following types of herbicides (b1-b18):
b1) inhibition of acetyl CoA carboxylase (inhibition of ACCase) herbicides;
b2) inhibition of acetolactate synthase (inhibition of ALS) or acetohydroxyacid synthase (inhibition of AHAS) herbicides;
b3) inhibition of photosynthesis at PSII herbicides;
b4) photosystem I electron diversion (PS I inhibitor) herbicides;
b5) inhibition of protoporphyrinogen oxidase (PPO inhibitor) herbicides;
b6) inhibition of phytoene desaturase (PDS inhibitor) herbicides;
b7) inhibition of hydroxyphenyl pyruvate dioxidase (HPPD inhibitor) herbicides;
b8) other pigment synthesis inhibitor herbicides;
b9) inhibition of enolpyruvyl shikimate phosphate synthase (inhibition of EPSP synthase) herbicides;
b10) inhibition of glutamine synthase (GS inhibitor) herbicides;
b11) inhibition of dihydropteroate synthase herbicides;
b12) inhibition of microtubule assembly and microtubule organization herbicides;
b13) inhibition of very long-chain fatty acid synthase herbicides;
b14) inhibition of cellulose synthesis herbicides;
b15) oxidative phosphorylation uncoupler herbicides;
b16) lipid synthesis (non-ACCase) inhibitor herbicides;
b17) auxin mimics herbicides.

The present invention relates to a herbicide composition comprising the active ingredient A and the active ingredient B. One skilled in the art can expect reasonably that a combination of the active ingredient A and the active ingredient B can achieve a good result.
b1) is selected from quizalofop-P-ethyl, haloxyfop-P-methyl, fluazifop-P-butyl, fenoxaprop-P, cyhalofop-butyl, quizalofop-P-tefuryl, quizalofop, haloxyfop-methyl, fenoxaprop, fluazifop-butyl, esprocarb, sethoxydim, cloproxydim, cycloxydim, clethodim, methoxyphenone, butroxydim, tepraloxydim, profoxydim, clodinafop-propargyl, pinoxaden, metamifop, propaquizafop, tralkoxydim;
b2) is selected from metsulfuron-methyl, chlorimuron, pyrazosulfuron, thifensulfuron, cinosulfuron, ethametsulfuron, cyclosulfamuron, ethoxysulfuron, rimsulfuron, primisulfuron, mesosulfuron-methyl, azimsulfuron, bensulfuron, chlorsulfuron, flazasulfuron, flucetosulfuron, foramsulfuron, halosulfuron, imazosulfuron, iodosulfuron-methyl-sodium, nicosulfuron, orthosulfamuron, oxasulfuron, prosulfuron, sulfosulfuron, triasulfuron, tribenuron, trifloxysulfuron, triflusulfuron, monosulfuron, iofensulfuron, tritosulfuron, propyrisulfuron, imazamethabenz, imazapic, imazapyr, imazaquin, flumetsulam, florasulam, metosulam, penoxsulam, pyroxsulam, bispyribac, pyribenzoxim, pyriftalid, pyriminobac-methyl, flucarbazone-sodium, propoxycarbazone-sodium, triafamone, imazethapyr, imazamox, cloransulam-methyl, diclosulam, bispyribac-sodium, pyrithiobac-sodium;
b3) is selected from atrazine, prometryn, metribuzin, terbutryn, terbuthylazine, cyanazine, propazine, simazine, ametryn, simetryn, amicarbazone, isoproturon, chlorotoluro, diuron, daimuron, propanil, bentazone, bromoxynil octanoate, pyridate, bromacil, lenacil, terbacil, desmedipham, phenmedipham, bromoxynil, ioxynil;
b4) is selected from paraquat, diquat;
b5) is selected from saflufenacil, carfentrazone-ethyl, flumioxazin, fomesafen, fluoroglycofen, acifluorfen, oxyfluorfen, lactofen, oxadiazon, oxadiargyl, flumiclorac, sulfentrazone, pyraclonil, pentoxadiazon, butafenacil, fluthiacet-methyl, azafenidin, benzfendizone, bifenox, chlomethoxynil, cinidon-ethyl, fluazolate, flufenpyr-ethyl, flupoxam, flumiclorac-pentyl, tiafenacil, trifludimoxazin, aclonifen, cyclopyranil, epyrifenacil, flufenoximacil, KAI-162573(CAS NO.:2669821-71-8), SY-1604(CAS NO.:1949837-17-5), ethyl(5S)-3-(2-chloro-4-fluoro-5-(3-methyl-2,6-dioxo-4-(trifluoromethyl)-3,6-dihydropyrimidin-1(2H)-yl)phenyl)-5-methyl-4,5-dihydroisoxazole-5-carboxylate, ethyl(5R)-3-(2-chloro-4-fluoro-5-(3-methyl-2,6-dioxo-4-(trifluoromethyl)-3,6-dihydropyrimidin-1(2H)-yl)phenyl)-5-methyl-4,5-dihydroisoxazole-5-carboxylate;
b6) is selected from diflufenican, flurochloridone, flurtamone, norflurazon, fluridone, beflubutamid-M;
b7) is selected from mesotrione, tembotrione, benzobicyclon, tefuryltrione, lancotrione, sulcotrione, bicyclopyrone, topramezone, pyrasulfotole, benzofenap, pyrazolynate, pyrazoxyfen, tolpyralate, isoxaflutole, cypyrafluone, tripyrasulfone, flusulfinam, bipyrazone, fenpyrazone, fenquinotrione, benquitrione, Y13287(CAS NO.: 1639426-42-8), pyraquinate;
b8) is selected from clomazone, bixlozone, broclozone, beflubufamid, diflufenican, fluometuron;
b9) is selected from glyphosate;
b10) is selected from glufosinate-ammonium, glufosinate-P, bilanafos;
b11) is selected from asulam;
b12) is selected from butralin, trifluralin, dithiopyr, carbetamide, bethrodine, ethalfluralin, fluchloralin, oryzalin, pendimethalin, prodiamine, amiprophos, amiprofos-methyl, dimethyl tetrachloroterephthalate, propyzamide, carbetamide, chlorpropham, flamprop-methyl, flamprop-M-isopropyl, propham;
b13) is selected from butachlor, pretilachlor, mefenacet, anilofos, flufenacet, pyroxasulfone, s-metolachlor, acetochlor, piperophos, alachlor, amidochlor, ethachlor, thenylchlor, dimethenamid-P, metazachlor, metolachlor, pethoxamid, propachlor ESA sodium salt, propisochlor, mefenacet, diphenamid, naproanilide, devrinol, fentrazamide, cafenstrole, napropamide, fenoxasulfone, ipfencarbazone, indaziflam, methiozolin, napropamide-M;
b14) is selected from chlorthiamide, dichlobenil, flupoxan, indaziflam, isoxaben, triaziflam;
b15) is selected from dichlobenil, dinoterb;
b16) is selected from butylate, thiobencarb, vernolate, molinate, tri-allate, dimepiperate, asulam, cycloate, benfuresate, ethofumesate, 6-hydroxymelatonin, dalapon, EPTC, flupropanate, orbencarb, pebulate;
b17) is selected from 2,4-D, MCPA, 2,3,6-TBA, chloramben, dicamba, picloram, fluroxypyr, fluroxypyr-meptyl, quinclorac, quintrione, halauxifen-methyl, florpyrauxifen-benzyl, triclopyr, 3,6-Dichloropicolinic acid, quinmerac, clacyfos, aminocyclopyrachlor, benazolin, chloramben-methyl, clomeprop, quinmerac, diflufenzopyr, KAI-177022, fluchloraminopyr;
b18) is selected from cinmethylin, cyclopyrimorate, tetflupyrolimet, dimesulfazet, rimisoxafen, dioxopyritrione.

Preferably, a weight ratio of the active ingredient A to the active ingredient B in the herbicide composition is from 1:300 to 300:1; more preferably, a weight ratio of the active ingredient A to the active ingredient B in the herbicide composition is from 1:300 to 48:1; further preferably, a weight ratio of the active ingredient A to the active ingredient B in the herbicide composition is from 1:150 to 20:1.

In a preferred embodiment of the present invention, the weight ratio of the active ingredient A to the active ingredient B in the herbicide composition is from 1:300 to 3.3:1.

The weight percentage content of the active ingredient A and the active ingredient B in the herbicide composition is 1% to 95% of the total, preferably 10% to 80%.

The herbicide composition further comprises a conventional adjuvant, the adjuvant comprises a carrier and a surfactant.

The term "carrier" in the present invention refers to a substance being organic or inorganic, natural or synthetic, facilitating a use of the active ingredients. The carrier is generally inert and required to be agriculturally acceptable, especially by a plant to be treated. The carrier may be solid, such as clay, natural or synthetic silicate, silica, resin, wax, solid fertilizer, etc.; or liquid, such as water, alcohols, ketones, petroleum fractions, aromatic hydrocarbons or hydrocarbon waxes, chlorinated hydrocarbons, liquefied gases, etc.

The surfactant may include emulsifier, dispersant or wetting agent, which may be ionic or non-ionic. Examples that can be mentioned are salts of polyacrylic acid, lignosulfonates, salts of phenolsulfonic acid or naphthalenesulfonic acid, polymers of ethylene oxide, aliphatic alcohols (aliphatic acids or aliphatic amines) and substituted phenols (in particular alkyl phenols or aryl phenols), sulfosuccinates, taurine derivatives (in particular taurine alkyl ester) and phosphates of alcohols or polyhydroxyethylated phenols, alkyl sulfonates, alkyl aryl sulfonates, alkyl sulfates, laureth sulfates, fatty alcohol sulfates, and sulfated hexadecanol, heptadecanol and octadecanol, sulfated fatty alcohol glycol ether, furthermore, condensations of naphthalene or naphthalene sulfonic acid with phenol and formaldehyde, polyoxyethylene octylphenol ether, ethoxylated isooctylphenol, octylpheno, nonylphenol, alkylphenyl polyethylene glycol ether, tributylphenyl polyethylene glycol ether, tristearylphenyl polyethylene glycol ether, alkylaryl polyether alcohol, alcohol and fatty alcohol/oxidized ethylene condensate, ethoxylated castor oil, polyoxyethylene alkyl ether, ethoxylated polyoxypropylene, lauryl polyethylene glycol ether acetal, sorbitol ester, lignin sulfite waste, as well as protein, denatured protein, polysaccharide (e.g. methylcellulose), hydrophobic modified starch, polyvinyl alcohol, polycarboxylates, polyalkoxide, polyvinylamine, polyvinylpyrrolidone and their copolymers. At least one surfactant is required to be present to facilitate a dispersion of the active components in water thereby facilitating a correct use to a plant.

The above composition may further contain a variety of other components such as protective colloid, adhesive, thickener, thixotropic agent, penetrant, stabilizer, chelating agent, dye, colorant and polymer.

The composition of the present invention can be diluted before using or directly applied by a user. A formulation can be prepared by a conventional processing method, that is, mixing the active substance with a liquid solvent or a solid carrier, then adding one or more surfactants such as dispersant, stabilizer, humectant, adhesive, defoamer, etc.

The specific preparation of the herbicidal composition can be oil dispersion, suspension concentrate, suspension emulsion, wettable powder, emulsifiable concentrate, water dispersible granule (a dry flowable), water emulsion, microemulsion.

In short, the composition of the present invention may be mixed with a conventional solid or liquid additive used in the prior art. Following an external condition change, the amount of the effective ingredient applied also varies. The external condition comprises temperature, humidity, the property of the herbicide used, etc. The amount can have a large variation, such as from 0.001 to 1.0 kg/ha, or more active ingredients, preferably from 0.005 to 750 g/ha, particularly from 0.005 to 500 g/ha.

The present invention further provides a use of the herbicide composition in controlling weeds in useful crops, further, in useful crops of genetically modified crops or crops treated by genome editing technology, and furthermore, in controlling and killing annual or perennial weeds, resistant or tolerant weeds in crop cultivated land, garden land or non-cultivated land.

The present invention further provides a method for controlling the growth of unwanted plants, comprising applying the herbicide composition to a plant, a plant part, a plant seed or a plant growth area, further comprising a method of selectively controlling weeds in useful crops using the herbicide composition. The useful crops also comprise genetically modified crops or crops treated by genome editing technology.

In the context of the present description, if an abbreviated form of a common name of the active compound is used, all conventional derivatives, such as esters and salts, are included in each case, as well as isomers, especially optical isomers, in particular one or more commercially available forms. If the common name indicates esters or salts, all other conventional derivatives are also included in each case, such as other esters and salts, free acids and neutral compounds, as well as isomers, especially optical isomers, in particular one or more commercially available forms. The chemical name of the compound indicates at least one compound covered by the common name, usually the preferred compound. In the case of sulfonamides such as sulfonylureas, salts also include salts formed by the exchange of cations with hydrogen atoms in the sulfonamide group. For example, MCPA derivatives comprise, but not limited to: MCPA-sodium, MCPA-potassium, MCPA-dimethyl ammonium, MCPA-isopropylammonium, MCPA-methyl, MCPA-ethyl, MCPA-isoctyl, MCPA-thioethyl, etc.; 2.4-D derivatives comprise, but are not limited to: 2,4-D salts such as 2,4-D-sodium, 2,4-D-potassium, 2,4-D-dimethylammonium, 2,4-D-trolamine, 2,4-D-isopropylammonium, 2,4-D-choline, ect.; and 2,4-D esters such as 2,4-D-methyl, 2,4-D-ethyl, 2,4-D-butyl, 2,4-D-isoctyl, etc.

A preferred compound of formula (I), wherein each variable has the following meaning independently or in combination with each other.

### Table A Some compounds of the active ingredient A

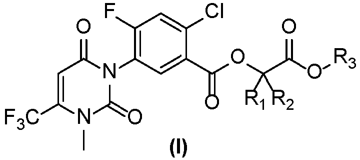

**Table A**

| Compound No. | R₁ | R₂ | R₃ |
|---|---|---|---|
| A-001 | H | H | CH₃ |
| A-002 | H | H | CH₂CH₃ |
| A-003 | H | H | CH₂CH₂CH₃ |
| A-004 | H | H | CH(CH₃)₂ |
| A-005 | H | H | CH₂CH₂CH₂CH₃ |
| A-006 | H | H | CH₂CH(CH₃)₂ |
| A-007 | H | H | C(CH₃)₃ |
| A-008 | H | H | 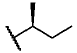 |
| A-009 | H | H | 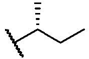 |
| A-010 | H | H | 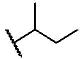 |
| A-011 | H | H | 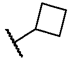 |
| A-012 | H | H | 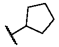 |
| A-013 | H | H | 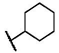 |
| A-014 | H | H | 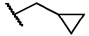 |
| A-015 | H | H | 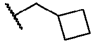 |
| A-016 | H | H | 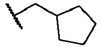 |
| A-017 | H | H | 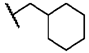 |
| A-018 | H | H | 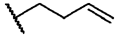 |
| A-019 | H | H | 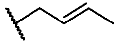 |
| A-020 | H | H | 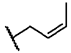 |
| A-021 | H | H | 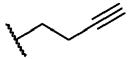 |
| A-022 | H | H | 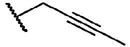 |
| A-023 | H | H | 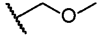 |
| A-024 | H | H | 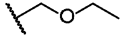 |
| A-025 | H | H | 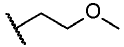 |
| A-026 | H | H | 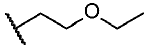 |
| A-027 | H | H | 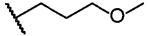 |
| A-028 | H | H | 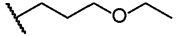 |
| A-029 | H | H | 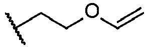 |
| A-030 | H | H | 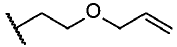 |
| A-031 | H | H | 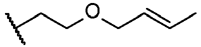 |
| A-032 | H | H | 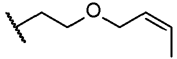 |
| A-033 | H | H | 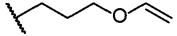 |
| A-034 | H | H | 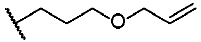 |
| A-035 | H | H | 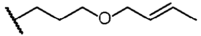 |
| A-036 | H | H | 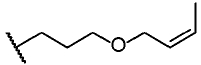 |
| A-037 | H | H | 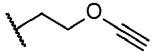 |
| A-038 | H | H | 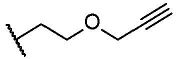 |
| A-039 | H | H | 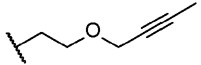 |
| A-040 | H | H | 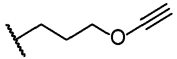 |
| A-041 | H | H | 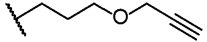 |
| A-042 | H | H | 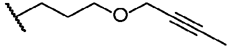 |
| A-043 | H | H | 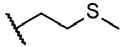 |
| A-044 | H | H | 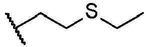 |
| A-045 | H | H | 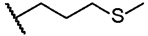 |
| A-046 | H | H | 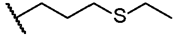 |
| A-047 | H | H | 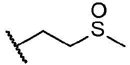 |
| A-048 | H | H | 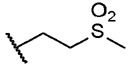 |
| A-049 | H | H | 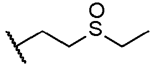 |
| A-050 | H | H | 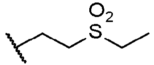 |
| A-051 | H | H | 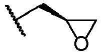 |
| A-052 | H | H | 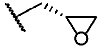 |
| A-053 | H | H | 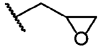 |
| A-054 | H | H | 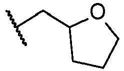 |
| A-055 | H | H | 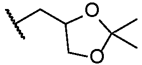 |
| A-056 | H | H | 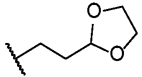 |
| A-057 | H | H | 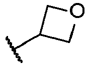 |
| A-058 | H | H | 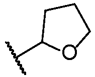 |
| A-059 | H | H | 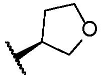 |
| A-060 | H | H | 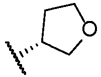 |
| A-061 | H | H | 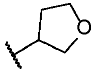 |
| A-062 | H | H | 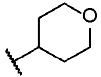 |
| A-063 | H | 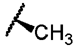 | CH₃ |
| A-064 | H | 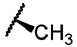 | CH₂CH₃ |
| A-065 | H | 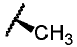 | CH₂CH₂CH₃ |
| A-066 | H | 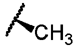 | CH(CH₃)₂ |
| A-067 | H | 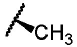 | CH₂CH₂CH₂CH₃ |
| A-068 | H | 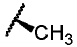 | CH₂CH(CH₃)₂ |
| A-069 | H | 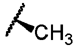 | C(CH₃)₃ |
| A-070 | H | 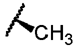 | 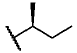 |
| A-071 | H | 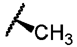 | 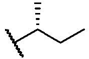 |
| A-072 | H | 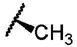 | 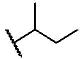 |
| A-073 | H | 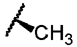 | 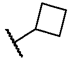 |
| A-074 | H | 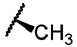 | 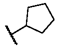 |
| A-075 | H | 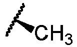 | 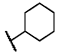 |
| A-076 | H | 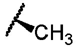 | 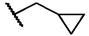 |
| A-077 | H | 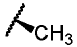 | 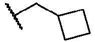 |
| A-078 | H | 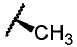 | 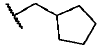 |
| A-079 | H | 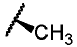 | 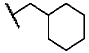 |
| A-080 | H | 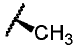 | 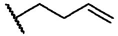 |
| A-081 | H | 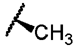 | 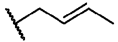 |
| A-082 | H | 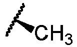 | 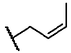 |
| A-083 | H | 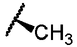 | 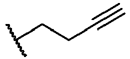 |
| A-084 | H | 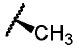 | 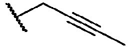 |
| A-085 | H | 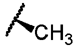 | 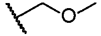 |
| A-086 | H | 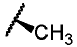 | 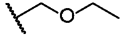 |
| A-087 | H | 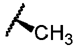 | 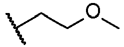 |
| A-088 | H | 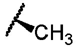 | 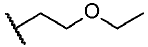 |
| A-089 | H | 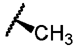 | 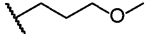 |
| A-090 | H | 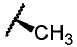 | 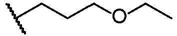 |
| A-091 | H | 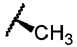 | 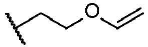 |
| A-092 | H | 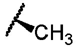 | 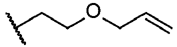 |
| A-093 | H | 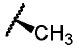 | 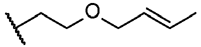 |
| A-094 | H | 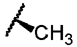 | 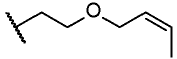 |
| A-095 | H | 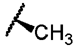 | 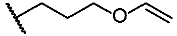 |
| A-096 | H | 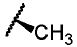 | 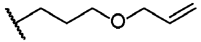 |
| A-097 | H | 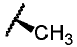 | 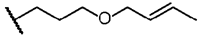 |
| A-098 | H | 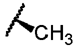 | 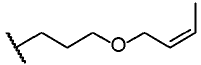 |
| A-099 | H | 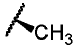 | 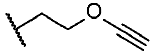 |
| A-100 | H | 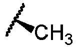 | 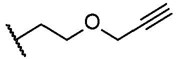 |
| A-101 | H | 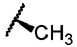 | 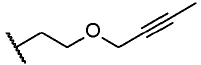 |
| A-102 | H | 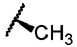 | 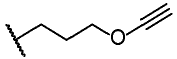 |
| A-103 | H | 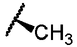 | 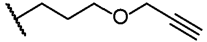 |
| A-104 | H | 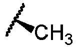 | 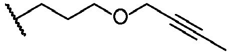 |
| A-105 | H | 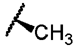 | 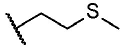 |
| A-106 | H | 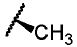 | 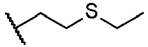 |
| A-107 | H | 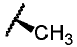 | 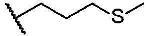 |
| A-108 | H | 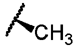 | 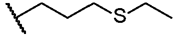 |
| A-109 | H | 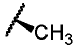 | 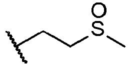 |
| A-110 | H | 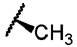 | 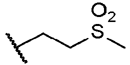 |
| A-111 | H | 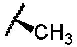 | 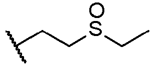 |
| A-112 | H | 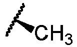 | 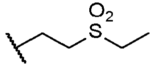 |
| A-113 | H | 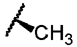 | 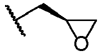 |
| A-114 | H | 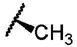 | 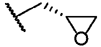 |
| A-115 | H | 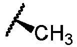 | 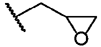 |
| A-116 | H | 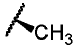 | 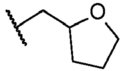 |
| A-117 | H | 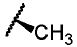 | 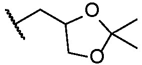 |
| A-118 | H | 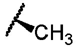 | 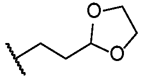 |
| A-119 | H | 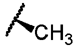 | 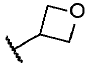 |
| A-120 | H | 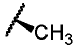 | 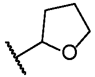 |
| A-121 | H | 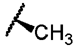 | 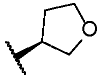 |
| A-122 | H | 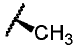 | 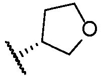 |
| A-123 | H | 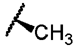 | 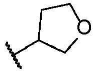 |
| A-124 | H | 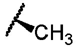 | 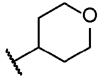 |
| A-125 | H | 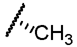 | CH₃ |
| A-126 | H | 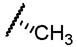 | CH₂CH₃ |
| A-127 | H | 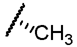 | CH₂CH₂CH₃ |
| A-128 | H | 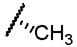 | CH(CH₃)₂ |
| A-129 | H | 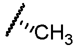 | CH₂CH₂CH₂CH₃ |
| A-130 | H | 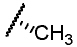 | CH₂CH(CH₃)₂ |
| A-131 | H | 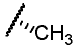 | C(CH₃)₃ |
| A-132 | H | 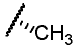 | 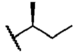 |
| A-133 | H | 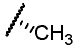 | 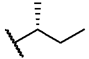 |
| A-134 | H | 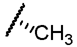 | 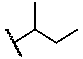 |
| A-135 | H | 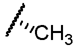 | 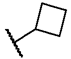 |
| A-136 | H | 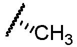 | 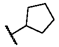 |
| A-137 | H | 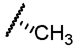 | 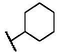 |
| A-138 | H | 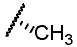 | 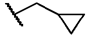 |
| A-139 | H | 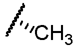 | 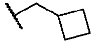 |
| A-140 | H | 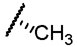 | 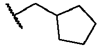 |
| A-141 | H | 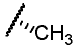 | 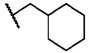 |
| A-142 | H | 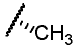 | 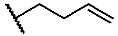 |
| A-143 | H | 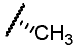 | 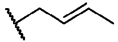 |
| A-144 | H | 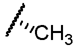 | 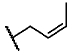 |
| A-145 | H | 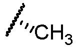 | 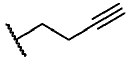 |
| A-146 | H | 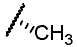 | 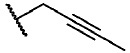 |
| A-147 | H | 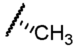 | 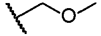 |
| A-148 | H | 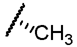 | 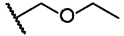 |
| A-149 | H | 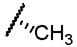 | 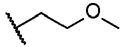 |
| A-150 | H | 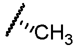 | 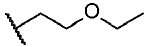 |
| A-151 | H | 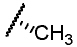 | 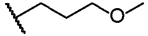 |
| A-152 | H | 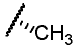 | 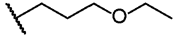 |
| A-153 | H | 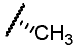 | 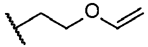 |
| A-154 | H | 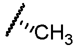 | 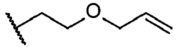 |
| A-155 | H | 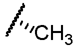 | 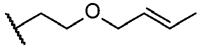 |
| A-156 | H | 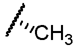 | 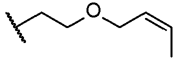 |
| A-157 | H | 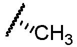 | 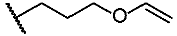 |
| A-158 | H | 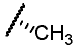 | 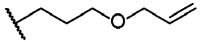 |
| A-159 | H | 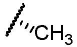 | 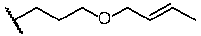 |
| A-160 | H | 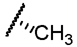 | 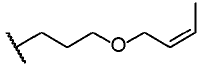 |
| A-161 | H | 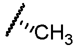 | 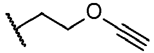 |
| A-162 | H | 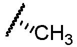 | 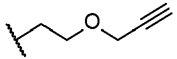 |
| A-163 | H | 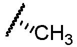 | 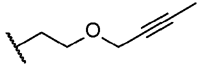 |
| A-164 | H | 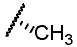 | 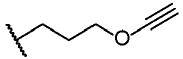 |
| A-165 | H | 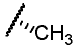 | 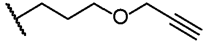 |
| A-166 | H | 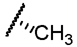 | 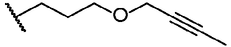 |
| A-167 | H | 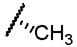 | 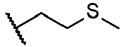 |
| A-168 | H | 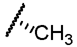 | 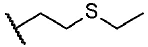 |
| A-169 | H | 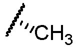 | 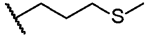 |
| A-170 | H | 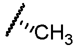 | 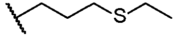 |
| A-171 | H | 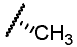 | 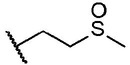 |
| A-172 | H | 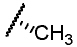 | 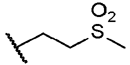 |
| A-173 | H | 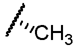 | 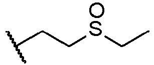 |
| A-174 | H | 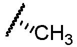 | 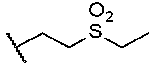 |
| A-175 | H | 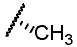 | 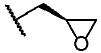 |
| A-176 | H | 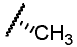 | 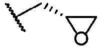 |
| A-177 | H | 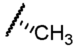 | 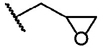 |
| A-178 | H | 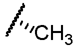 | 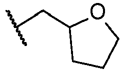 |
| A-179 | H | 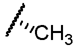 | 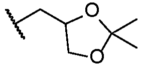 |
| A-180 | H | 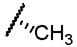 | 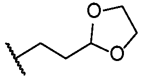 |
| A-181 | H | 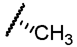 | 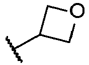 |
| A-182 | H | 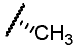 | 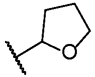 |
| A-183 | H | 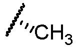 | 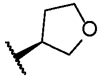 |
| A-184 | H | 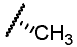 | 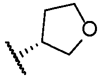 |
| A-185 | H | 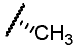 | 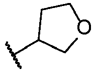 |
| A-186 | H | 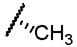 | 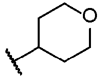 |
| A-187 | CH₃ | H | CH₃ |
| A-188 | CH₃ | H | CH₂CH₃ |
| A-189 | CH₃ | H | CH₂CH₂CH₃ |
| A-190 | CH₃ | H | CH(CH₃)₂ |
| A-191 | CH₃ | H | CH₂CH₂CH₂CH₃ |
| A-192 | CH₃ | H | CH₂CH(CH₃)₂ |
| A-193 | CH₃ | H | C(CH₃)₃ |
| A-194 | CH₃ | H | 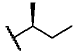 |
| A-195 | CH₃ | H | 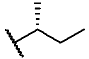 |
| A-196 | CH₃ | H | 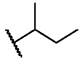 |
| A-197 | CH₃ | H | 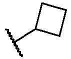 |
| A-198 | CH₃ | H | 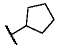 |
| A-199 | CH₃ | H | 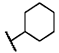 |
| A-200 | CH₃ | H | 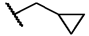 |
| A-201 | CH₃ | H | 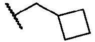 |
| A-202 | CH₃ | H | 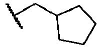 |
| A-203 | CH₃ | H | 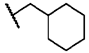 |
| A-204 | CH₃ | H | 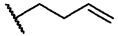 |
| A-205 | CH₃ | H | 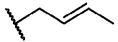 |
| A-206 | CH₃ | H | 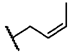 |
| A-207 | CH₃ | H | 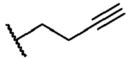 |
| A-208 | CH₃ | H | 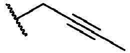 |
| A-209 | CH₃ | H | 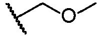 |
| A-210 | CH₃ | H | 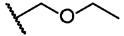 |
| A-211 | CH₃ | H | 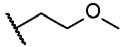 |
| A-212 | CH₃ | H | 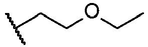 |
| A-213 | CH₃ | H | 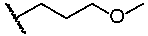 |
| A-214 | CH₃ | H | 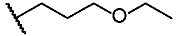 |
| A-215 | CH₃ | H | 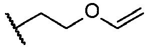 |
| A-216 | CH₃ | H | 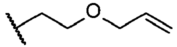 |
| A-217 | CH₃ | H | 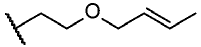 |
| A-218 | CH₃ | H | 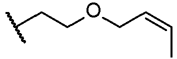 |
| A-219 | CH₃ | H | 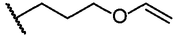 |
| A-220 | CH₃ | H | 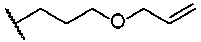 |
| A-221 | CH₃ | H | 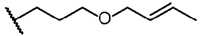 |
| A-222 | CH₃ | H | 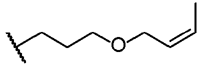 |
| A-223 | CH₃ | H | 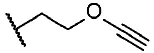 |
| A-224 | CH₃ | H | 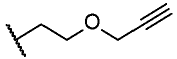 |
| A-225 | CH₃ | H | 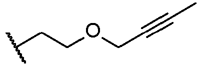 |
| A-226 | CH₃ | H | 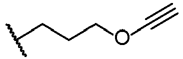 |
| A-227 | CH₃ | H | 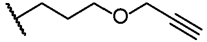 |
| A-228 | CH₃ | H | 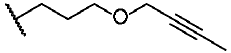 |
| A-229 | CH₃ | H | 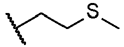 |
| A-230 | CH₃ | H | 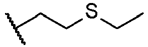 |
| A-231 | CH₃ | H | 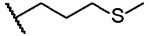 |
| A-232 | CH₃ | H | 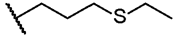 |
| A-233 | CH₃ | H | 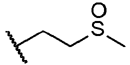 |
| A-234 | CH₃ | H | 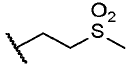 |
| A-235 | CH₃ | H | 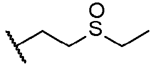 |
| A-236 | CH₃ | H | 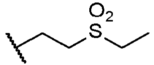 |
| A-237 | CH₃ | H | 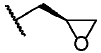 |
| A-238 | CH₃ | H | 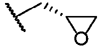 |
| A-239 | CH₃ | H | 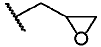 |
| A-240 | CH₃ | H | 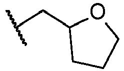 |
| A-241 | CH₃ | H | 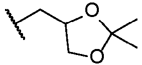 |
| A-242 | CH₃ | H | 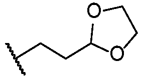 |
| A-243 | CH₃ | H | 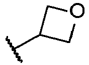 |
| A-244 | CH₃ | H | 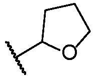 |
| A-245 | CH₃ | H | 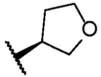 |
| A-246 | CH₃ | H | 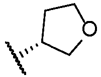 |
| A-247 | CH₃ | H | 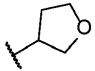 |
| A-248 | CH₃ | H | 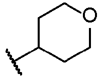 |
| A-249 | CH₃ | CH₃ | CH₃ |
| A-250 | CH₃ | CH₃ | CH₂CH₃ |
| A-251 | CH₃ | CH₃ | CH₂CH₂CH₃ |
| A-252 | CH₃ | CH₃ | CH(CH₃)₂ |
| A-253 | CH₃ | CH₃ | CH₂CH₂CH₂CH₃ |
| A-254 | CH₃ | CH₃ | CH₂CH(CH₃)₂ |
| A-255 | CH₃ | CH₃ | C(CH₃)₃ |
| A-256 | CH₃ | CH₃ | 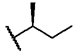 |
| A-257 | CH₃ | CH₃ | 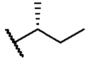 |
| A-258 | CH₃ | CH₃ | 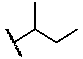 |
| A-259 | CH₃ | CH₃ | 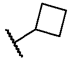 |
| A-260 | CH₃ | CH₃ | 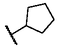 |
| A-261 | CH₃ | CH₃ | 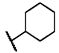 |
| A-262 | CH₃ | CH₃ | 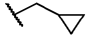 |
| A-263 | CH₃ | CH₃ | 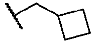 |
| A-264 | CH₃ | CH₃ | 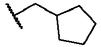 |
| A-265 | CH₃ | CH₃ | 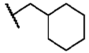 |
| A-266 | CH₃ | CH₃ | 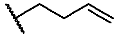 |
| A-267 | CH₃ | CH₃ | 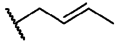 |
| A-268 | CH₃ | CH₃ | 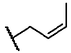 |
| A-269 | CH₃ | CH₃ | 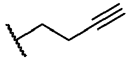 |
| A-270 | CH₃ | CH₃ | 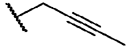 |
| A-271 | CH₃ | CH₃ | 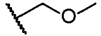 |
| A-272 | CH₃ | CH₃ | 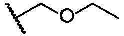 |
| A-273 | CH₃ | CH₃ | 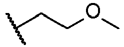 |
| A-274 | CH₃ | CH₃ | 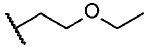 |
| A-275 | CH₃ | CH₃ | 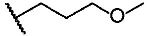 |
| A-276 | CH₃ | CH₃ | 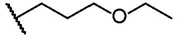 |
| A-277 | CH₃ | CH₃ | 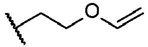 |
| A-278 | CH₃ | CH₃ | 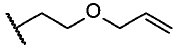 |
| A-279 | CH₃ | CH₃ | 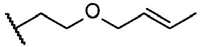 |
| A-280 | CH₃ | CH₃ | 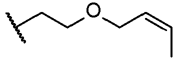 |
| A-281 | CH₃ | CH₃ | 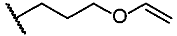 |
| A-282 | CH₃ | CH₃ | 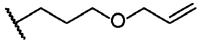 |
| A-283 | CH₃ | CH₃ | 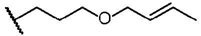 |
| A-284 | CH₃ | CH₃ | 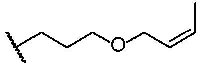 |
| A-285 | CH₃ | CH₃ | 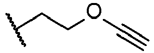 |
| A-286 | CH₃ | CH₃ | 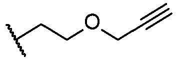 |
| A-287 | CH₃ | CH₃ | 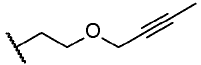 |
| A-288 | CH₃ | CH₃ | 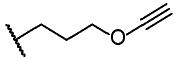 |
| A-289 | CH₃ | CH₃ | 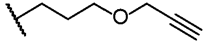 |
| A-290 | CH₃ | CH₃ | 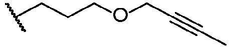 |
| A-291 | CH₃ | CH₃ | 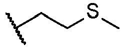 |
| A-292 | CH₃ | CH₃ | 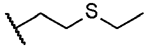 |
| A-293 | CH₃ | CH₃ | 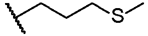 |
| A-294 | CH₃ | CH₃ | 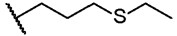 |
| A-295 | CH₃ | CH₃ | 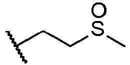 |
| A-296 | CH₃ | CH₃ | 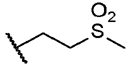 |
| A-297 | CH₃ | CH₃ | 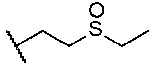 |
| A-298 | CH₃ | CH₃ | 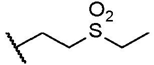 |
| A-299 | CH₃ | CH₃ | 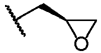 |
| A-300 | CH₃ | CH₃ | 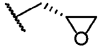 |
| A-301 | CH₃ | CH₃ | 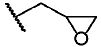 |
| A-302 | CH₃ | CH₃ | 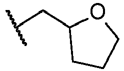 |
| A-303 | CH₃ | CH₃ | 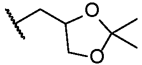 |
| A-304 | CH₃ | CH₃ | 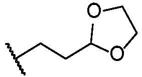 |
| A-305 | CH₃ | CH₃ | 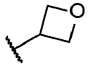 |
| A-306 | CH₃ | CH₃ | 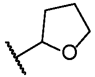 |
| A-307 | CH₃ | CH₃ | 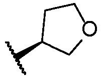 |
| A-308 | CH₃ | CH₃ | 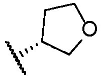 |
| A-309 | CH₃ | CH₃ | 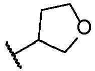 |
| A-310 | CH₃ | CH₃ | 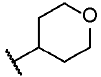 |

The ingredient B is selected from herbicide B as defined in b1)-b18) above, in particular the herbicides listed in the table below:

**Table B**

| NO. | Herbicide B | No. | Herbicide B | No. | Herbicide B |
|---|---|---|---|---|---|
| B1 | | B59 | Profoxydim | B117 | Sethoxydim |
| B2 | Picloram | B60 | Cypyrafluone | B118 | Mesotrione |
| B3 | Amicarbazone | B61 | Cyclosulfamuron | B119 | Bromoxynil octanoate |
| B4 | Flupoxan | B62 | Aminocyclopyrachlor | B120 | Nicosulfuron |
| B5 | Paraquat | B63 | Cinmethylin | B121 | tri-allate |
| B6 | Tribenuron | B64 | Tembotrione | B122 | Acetochlor |
| B7 | Saflufenacil | B65 | Sulcotrione | B123 | Fluoroglycofen |
| B8 | Topramezone | B66 | Alachlor | B124 | Oxyfluorfen |
| B9 | Fenpyrazone | B67 | Mesosulfuron-methyl | B125 | S-metolachlor |
| B10 | Diflufenican | B68 | | B126 | Isoproturon |
| B11 | Pyrazosulfuron | B69 | Imazapic | B127 | Clomazone |
| B12 | Pyraquinate | B70 | Foramsulfuron | B128 | Isoxaflutole |
| B13 | | B71 | Imazamox | B129 | Atrazine |
| B14 | Bensulfuron | B72 | Fenoxaprop-P-ethyl | B130 | Butralin |
| B15 | Pretilachlor | B73 | Quizalofop-P-Ethyl | B131 | Carfentrazone-ethyl |
| B16 | Oxadiargyl | B74 | Dimethenamid-P | B132 | Pinoxaden |
| B17 | Flumioxazin | B75 | S-metolachlor | B133 | Flumetsulam |
| B18 | Glufosinate-ammonium | B76 | Benquitrione | B134 | Azimsulfuron |
| B19 | Benazolin | B77 | Chlorotoluron | B135 | 2,4-D |
| B20 | | B78 | Halosulfuron | B136 | MCPA |
| B21 | Glyphosate | B79 | florpyrauxifen-benzyl | B137 | Bixlozone |
| B22 | Monosulfuron | B80 | Fluroxypyr | B138 | Cloproxydim |
| B23 | Dichlobenil | B81 | Chlorimuron | B139 | Cyclopyranil |
| B24 | Propanil | B82 | Clacyfos | B140 | Cyclopyrimorate |
| B25 | Napropamide | B83 | Dicamba | B141 | Dimesulfazet |
| B26 | Diquat | B84 | | B142 | Dioxopyritrione |
| B27 | Diuron | B85 | Imazethapyr | B143 | Epyrifenacil |
| B28 | | B86 | Pyribenzoxim | B144 | Fenoxasulfone |
| B29 | Butachlor | B87 | Bentazone | B145 | Fenquinotrione |
| B30 | Butylate | B88 | | B146 | Indaziflam |
| B31 | Pyroxsulam | B89 | Prometryn | B147 | Iofensulfuron |
| B32 | Flazasulfuron | B90 | | B148 | Ipfencarbazone |
| B33 | Oxadiazon | B91 | Metribuzin | B149 | Lancotrione |
| B34 | Fenoxaprop | B92 | Cyanazine | B150 | Methiozolin |
| B35 | Metamifop | B93 | Cyhalofop-butyl | B151 | Napropamide-M |
| B36 | Pendimethalin | B94 | Clodinafop-propargyl | B152 | Pyrasulfotole |
| B37 | 3,6-Dichloropicolinic acid | B95 | Lactofen | B153 | Rimisoxafen |
| B38 | Quinclorac | B96 | Cycloxydim | B154 | Tetflupyrolimet |
| B39 | Pyroxasulfone | B97 | Thifensulfuron | B155 | Tiafenacil |
| B40 | Flurtamone | B98 | | B156 | Tolpyralate |
| B41 | Tefuryltrione | B99 | Acifluorfen | B157 | Trifludimoxazin |
| B42 | Bicyclopyrone | B100 | Triclopyr | B158 | Sulfentrazone |
| B43 | Butafenacil | B101 | Tripyrasulfone | B159 | Y13287(CAS NO.: 1639426-42-8) |
| B44 | Beflubutamid | B102 | | B160 | Asulam |
| B45 | Trifloxysulfuron | B103 | Anilofos | B161 | Mefenacet |
| B46 | Flusulfinam | B104 | Bispyribac-sodium | B162 | Flufenacet |
| B47 | Fluorochloridone | B105 | Florasulam | B163 | Triaziflam |
| B48 | Fomesafen | B106 | Benzobicyclon | B164 | Ethofumesate |
| B49 | Trifluralin | B107 | Pyraclonil | B165 | |
| B50 | Dithiopyr | B108 | Bipyrazone | B166 | Flufenoximacil |
| B51 | Fluchloraminopyr | B109 | Terbutylazine | B167 | Fluchloraminopyr |
| B52 | halauxifen-methyl | B110 | Terbutryn | B168 | KAI-162573(CAS NO.:2669821-71-8) |
| B53 | Triafamone | B111 | Phenmedipham | B169 | SY-1604(CAS NO.: 1949837-17-5) |
| B54 | | B112 | Tralkoxydim | B170 | Glufosinate-P |
| B55 | flucarbazone-sodium | B113 | Penoxsulam | B171 | KAI-177022 |
| B56 | Haloxyfop-P-methyl | B114 | Simetryne | B172 | Broclozone |
| B57 | Thiobencarb | B115 | Simazine | B173 | Bialaphos |
| B58 | Esprocarb | B116 | Clethodim | | |

When having ionizable functional groups, the active ingredient A and the active ingredient B above can also be used in a form of an agricultural salt. A suitable salt is usually a cationic salt with a cation and an anion thereof having no adverse effect on an activity of the active compound respectively, and an acid addition salt of an acid.

A preferred cation is an ion of an alkali metal, preferably lithium ion, sodium ion and potassium ion, an ion of an alkaline earth metal, preferably calcium ion and magnesium ion, and an ion of a transition metal, preferably manganese ion, copper ion, zinc ion and iron ion, in addition, an ammonium ion and a substituted ammonium ion having 1-4 hydrogen atoms substituted by C₁-C₄ alkyl, hydroxy-C₁-C₄ alkyl, C₁-C₄ alkoxy-C₁-C₄ alkyl, hydroxy-C₁-C₄ alkoxy-C₁-C₄-alkyl, phenyl or benzyl, preferably ammonium, methylammonium, isopropylammonium, dimethylammonium, diisopropylammonium, trimethylammonium, heptylammonium, dodecylammonium, tetradecylammonium, tetramethylammonium, tetraethylammonium, tetrabutylammonium, 2-hydroxyethylammonium (alcoholamine salt), 2-(2-hydroxyethyl-1-oxy)ethyl-1-ammonium (diethylene glycolamine salt), bis(2-hydroxyethyl-1-yl) ammonium (diolamine salt), tris(2-hydroxyethyl) ammonium (triolamine salt), tris(2-hydroxypropyl) ammonium, benzyltrimethylammonium, benzyl triethylammonium, N,N,N-trimethylethanolammonium (choline salt); in addition, one more ion as a sulfonium ion, preferably tris(C₁-C₄ alkyl) sulfonium, such as trimethylsulfonium, and sulfoxonium ions, preferably tris(C₁-C₄ alkyl) sulfoxonium, and polyamines such as salts of N,N-bis(3-aminopropyl)methylamine and diethylenetriamine.

The anions of the acid addition salts are mainly chloride ion, bromine ion, fluoride ion, iodine ion, hydrogensulfate, methyl sulfate, sulfate, dihydrogen phosphate, hydrogen phosphate, nitrate, bicarbonate, carbonate, hexafluorosilicate, hexafluorophosphate, benzoate, and also C₁-C₄ alkanic acid anions, preferably formate, acetate, propionate and butyrate.

Alternatively, the ingredient A and the ingredient B components may also be in the form of acids, agricultural salts as described above, or agricultural derivatives, such as amide, for example mono- and di-C₁-C₆ alkyl amide or aryl amide, such as ester, for example allyl ester, propargyl ester, C₁-C₁₀ alkyl ester, alkoxyalkyl ester, tetrahydrofurylmethyl ((tetrahydrofuran-2-yl) methyl) ester, and also such as thioester, for example C₁-C₁₀ alkthioester. Preferred mono- and di-C₁-C₆ alkyl amide are methylamide and dimethylamide. Preferred arylamide, for example, anilid and 2-chloroacetanilide. Preferred alkyl esters such as methyl, ethyl, propyl, isopropyl, butyl, isobutyl, pentyl, methylhexyl (1-methylhexyl), methylheptyl (1-methylheptyl), heptyl, octyl or isooctyl (2-ethylhexyl) esters.

The present invention further provides a herbicide composition that can be applied as a herbicide, wherein the herbicide composition acts as an effective component, and the weight percentage content of the effective component is 1~99%. The herbicide preparation may be water emulsion, oil dispersion, microemulsion, emulsifiable concentrate, soluble concentrate, aqueous agent, suspension agent, suspension emulsion, ultra-low volume agent, powder, wettable powder, granule, water dispersible granule, dry flowable, microcapsule suspension, microcapsule granule, effervescent granule, effervescent tablet, water-dispersible tablet. The composition of the present invention may also have surfactants including emulsifier, dispersant, wetting agent, to increase the stability, permeability and wettability of the composition.

The dispersant suitable for preparing the composition of the present invention may be one or more of naphthalene or alkylnaphthalene formaldehyde condensate sulfonate, fatty alcohol ethylene oxide adduct sulfonate, alkylphenol polyoxyethylene ether sulfonate, polycarboxylate, fatty alcohol ethylene oxide adduct phosphate, alkylphenol polyoxyethylene ether formaldehyde condensate sulfate, polyoxyethylene polyoxypropylene ether block copolymer, alkylphenol polyoxyethylene phosphate, lignin sulfonate, gelatin, gum arabic, carboxymethylcellulose, polyoxyethylene alcohol, polyvinylpyrrolidone, sodium polyacrylate, polyethylene glycol.

The wetting agent suitable for preparing the composition of the present invention may be one or more of fatty alcohol sulfate, alkyl alcohol polyoxyethylene ether sodium sulfate, alkyl phenol polyoxyethylene ether sodium sulfate, alkyl phenol polyoxyethylene ether formaldehyde condensate sulfate, sodium alkyl sulfate, alkylnaphthalene sulfonate, fatty alcohol ethylene oxide adduct sulfonate, alkyl phenol formaldehyde condensation ethylene oxide adduct sulfonate, alkamidosulfonate, fatty alcohol polyoxyethylene ether, alkyl phenol polyoxyethylene ether, sorbitan fatty acid ester, sorbitan fatty acid ester polyoxyethylene ether, polyoxyethylene polyoxypropylene ether block copolymer, alkyl phenol formaldehyde condensation polyoxyethylene ether.

The solid carrier suitable for preparing the composition of the present invention may be: clay, kaolin, gypsum, bentonite, diatomaceous earth, talc, figuline, magnesium aluminum silicate, activated clay, silica, limestone, light calcium carbonate, corn starch, soluble starch, hexamethylene diammonium, benzofuran resin, styrene polymer and copolymer, etc.

The adhesive suitable for preparing the composition of the present invention may be natural or synthetic, such as xanthan gum, gelatin, gum arabic, polyvinylpyrrolidone, magnesium aluminum silicate, polyvinyl alcohol, phenolic resin, shellac, carboxymethylcellulose, methylcellulose and sodium alginate. The defoamer may be bubble enemy, silicone, C_{8~10} fatty alcohol, C_{10~20} saturated fatty acid and amide.

A disintegrant suitable for preparing the composition of the present invention may be one or more of calcium chloride, sodium sulfate, ammonium sulfate, ethyl polyacrylate, sodium carbonate, polyvinylpyrrolidone, sodium alginate, sodium carboxymethyl starch.

Examples for preparing representative compounds of the required active ingredient A are as follows, a synthesis method of other compounds is similar, and no more details are repeated herein.

If the name of a compound in the present invention is in conflict with the structural formula, the structural formula shall prevail unless the structural formula is obviously wrong.

A beneficial effect of the present invention is that the present invention provides a herbicide composition of a uracil compound containing a carboxylate fragment, and applying the herbicidal composition into practice as a herbicide has achieved satisfactory effect and selectivity. In addition, the preparation method and using method of the present invention are simple and easy.

### DETAILED DESCRIPTION OF THE INVENTION

Hereinafter, the present invention will be described with reference to the following but not limiting examples. Any simple replacement or improvement made to the present invention by one skilled in the art shall fall within the protection scope of the present invention.

Several methods for preparing the compounds of the present invention are explained in detail in the following schemes and examples. The raw materials can be purchased from the market or prepared using methods known in the literature or as detailed in the explanation. One skilled in the art should understand that the compounds of the present invention can also be synthesized using other synthesis routes. Although specific raw materials and conditions in the synthesis route have been explained below, they can be easily replaced with other similar raw materials and conditions, and various isomers of the compounds prepared by variants or variations of the preparation method of the present invention shall fall within the protection scope of the present invention. In addition, the preparation method described below can be further modified according to the disclosure of the present invention, using conventional chemical methods well-known to one skilled in the art, for example, protecting appropriate groups during the reaction process, and so on.

The method examples provided below are intended to promote further understanding of the preparation method of the present invention, and the specific substances, types, and conditions used are determined as further explanations of the present invention, not as limitations to the reasonable protection scope of the present invention. The raw materials and reagents used in the synthesis of the compounds described below can either be purchased from the market or easily prepared by one skilled in the art.

### Example 1: Preparation of intermediate 001-8

### Step 1: Preparation of intermediate 001-1

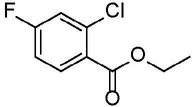

In a 500 mL four-necked flask, were placed 20 g 2-chloro-4-fluorobenzoic acid and 100 g of ethanol. The mixture was stirred and cooled to 0°C, and 17.73 g thionyl chloride was added dropwise slowly, while keeping the temperature below 0°C throughout the process. After completing adding thionyl chloride dropwise, the reaction solution was heated to 75 °C to reflux, reacted overnight while stirring, and spin-dried to obtain 23.01 g intermediate 001-1.

### Step 2: Preparation of intermediate 001-2

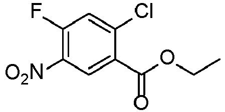

In a 1 L four-necked flask, were placed 67.46 g intermediate 001-1 and 337.3 g 1,2-dichloroethane, cooling down to 0°C, and 42.09 g fuming nitric acid (90%) and 60.12 g sulfuric acid (98%) were added dropwise slowly. After the addition, the mixture was heated slowly to room temperature and stirred until the reaction is completed. The reaction solution was transferred to a separating funnel, leaving statically until stratification to take the organic phase. The inorganic phase was extracted with 1,2-dichloroethane, and the acid in the organic phase was eluted with ice water until the pH value of the aqueous phase is about 7.0 and the organic phase was spin-dried to remove the solvent to obtain 89.81 g crude product. The crude product was recrystallized with n-hexane in 5 times of mass, filtering and drying a filter cake to obtain 42.45 g intermediate 001-2.

### Step 3: Preparation of intermediate 001-3

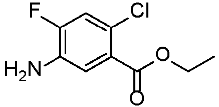

In a 1L autoclave, were placed 60.84 g intermediate 001-2, 4.87 g Pt/C (5%), 300 mL ethanol, while controlling the hydrogen pressure to 2MPa. After the mixture was reacted for 11 h at 45°C, Pt/C was removed by suction filtration, and the filtrate was spin-dried to obtain 52.48 g crude intermediate 001-3.

### Step 4: Preparation of intermediate 001-4

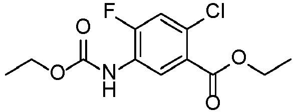

In a 500 mL four-necked flask, were placed 52.48 g crude intermediate 001-3, 24.78 g pyridine and 262.4 g dichloromethane while stirring at room temperature. After 5 min, 34.02 g ethyl chloroformate was diluted with 68.04 g dichloromethane, which was added dropwise slowly for 1 h. The mixture was reacted for 5 h, and the pH value was adjusted to be weakly acidic. The mixture was added with water and extracted by dichloromethane, and the organic phase was spin-dried to obtain 69.62 g crude intermediate 001-4.

### Step 5: Preparation of intermediate 001-5

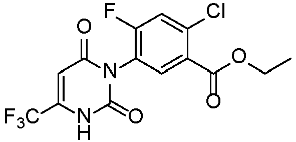

12.98 g sodium ethanol was dissolved in 38 g DMF while stirring, which was cooled to 5°C in ice bath, and added dropwise with DMF (28 g) solution of ethyl 3-amino-4,4,4-trifluorobutenoate (27.95 g) in ice bath. After addition, 36.84 g DMF solution of intermediate 001-4 was added dropwise. After addition, the mixture was heated to 100 °C and stirred for 5 h, after completing the reaction, the pH value was adjusted to acidic. The mixture was extracted with ethyl acetate, and the organic phase was washed with saturated saline, dried with anhydrous sodium sulfate and spin-dried to remove the solvent to obtain 50 g crude product, which was purified by column chromatography to obtain 19.05 g intermediate 001-5.

### Step 6: Preparation of intermediate 001-6

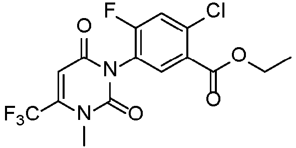

In a mono-necked flask, were placed 19.05 g intermediate 001-5 and 8.287 g anhydrous potassium carbonate which were dissolved with 60 g THF, 7.566 g dimethyl sulfate was added while stirring overnight at room temperature. After completing the reaction, the mixture was spin-dried to remove THF, extracted by ethyl acetate. The organic phase was dried by anhydrous sodium sulfate, and spin-dried to obtain 19.62 g crude intermediate 001-6.

### Step 7: Preparation of intermediate 001-7

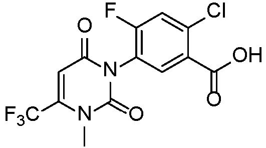

19.62 g intermediate 001-6 was dissolved in 150 mL acetic acid at room temperature, and 36 wt% hydrochloric acid of a same volume was added. The mixture was refluxed and reacted for 8 h, and distilled under reduced pressure to remove excess solvent after completing the reaction. The residue wad added with water to precipitate solid, stirred and filtered. The filter cake was washed with water for 3 times and dried at 60 °C to obtain 12.73 g crude intermediate 001-7.

### Step 8: Preparation of intermediate 001-8

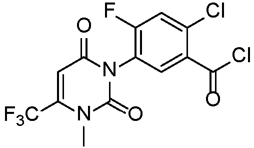

In a 100 mL mono-necked flask, were placed 6.8 g intermediate 001-7, 35 g 1,2-dichloroethane, 1 drop DMF and 3.316 g thionyl chloride. The mixture was refluxed and reacted for 3 h, and spin-dried to remove excess thionyl chloride and solvent after completing the reaction to obtain 6.22 g crude intermediate 001-8.

### Example 2: Preparation of compound A-002

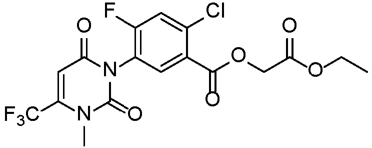

In a reaction flask, were placed 97.31 mg ethyl glycolate, 0.118 g triethylamine, 4.76 mg DMAP and 2 mL dichloromethane. The mixture was cooled in ice bath while blowing nitrogen, and 3 mL methylene chloride solution of intermediate 001-8 (0.30 g) as described in example 1 was added dropwise. After addition, the mixture was reacted for 2 h at room temperature, and purified by column chromatography after completing the reaction to obtain 240 mg compound A-002. ¹H NMR (400 MHz, DMSO-*d*₆) δ 8.16 (d, *J* = 7.8 Hz, 1H), 7.95 (d, *J =* 9.6 Hz, 1H), 6.61 (s, 1H), 4.95 (s, 2H), 4.19 (q, *J =* 7.1 Hz, 2H), 3.42 (s, 3H), 1.23 (d, *J =* 7.1 Hz, 3H). LCMS (ESI) [M + H]⁺=453.04, Found =453.31.

### Example 3: Preparation of compound A-188

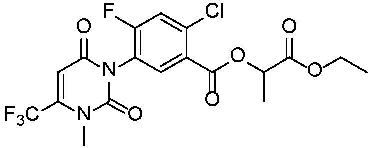

In a reaction flask, were placed 0.184 g ethyl lactate, 0.197 g triethylamine and 7.93 mg DMAP. The mixture was cooled in ice bath while stirring and blowing nitrogen, 3 mL dichloromethane solution of intermediate 001-8 (0.5 g) as described in the example 1 was added dropwise. After addition, the mixture was reacted for 2 h at room temperature, and purified by column chromatography after completing the reaction to obtain 290 mg compound A-188. ¹H NMR (400 MHz, DMSO-*d*₆) δ 8.20 (d, *J =* 7.7 Hz, 1H), 7.99 (d, *J =* 9.5 Hz, 1H), 6.68 (d, *J =* 3.6 Hz, 1H), 5.39 - 5.29 (m, 1H), 4.23 (dq, *J =* 7.1, 4.2 Hz, 2H), 3.48 (s, 3H), 1.59 (d, *J =* 7.0 Hz, 3H), 1.27 (t, *J =* 7.1 Hz, 3H). LCMS (ESI) [M + H]⁺=467.06, Found =466.98.

### Example 4: Preparation of compound A-250

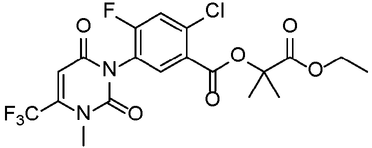

In a reaction flask, were placed 0.124 g ethyl 2-hydroxyisobutyrate, 0.114 g DMAP and 2 mL dichloromethane. The mixture was cooled in ice bath while blowing nitrogen, 3 mL of dichloromethane solution of intermediate 001-8 (0.30 g) described in example 1 was added dropwise. After addition, the mixture was reacted for 3 h at room temperature, and purified by column chromatography after completing the reaction to obtain 225 mg compound A-250. ¹H NMR (400 MHz, DMSO-*d*₆) δ 8.13 (d, *J =* 7.8 Hz, 1H), 7.97 (d, *J =* 9.6 Hz, 1H), 6.68 (s, 1H), 4.21 (q, *J =* 7.1 Hz, 2H), 3.48 (s, 3H), 1.68 (s, 6H), 1.24 (t, *J =* 7.1 Hz, 3H). LCMS (ESI) [M + H]⁺= 481.07, Found =481.25.

### Example 5: Preparation of compound A-025

### Step 1: Preparation of intermediate 025-1

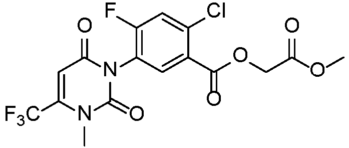

In a reaction flask, were placed intermediate 001-8 (1 g) as described in example 1 and 320 mg methyl glycolate. The mixture was cooled in ice bath while stirring and blowing nitrogen, and 394 mg triethylamine was added dropwise. After addition, the mixture was reacted for 2 h at the room temperature, and purified by column chromatography after completing the reaction to obtain 1.02 g intermediate 025-1.

### Step 2: Preparation of intermediate 025-2

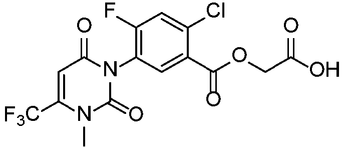

In a reaction flask, were placed 1.02 g intermediate 025-1, 6.12 g hydrochloric acid (36%), and 6.12 g acetic acid. The reaction solution was refluxed for 40 min, and spin-dried to obtain 1.01 g intermediate 025-2.

### Step 3: Preparation of intermediate 025-3

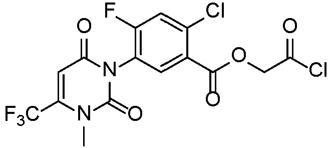

In a reaction flask, were placed 1.01 g intermediate 025-2, 340 mg thionyl chloride, 2 drops DMF, and 5.5 g dichloroethane. The reaction solution was refluxed, reacted for 3 h, and spin-dried to obtain 1.02 g intermediate 025-3.

### Step 4: Preparation of compound A-025

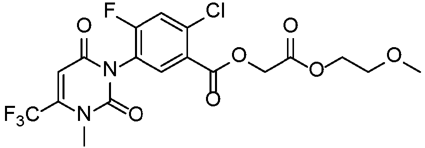

In a reaction flask, were placed 103.29 mg ethylene glycol methyl ether and 228.95 mg triethylamine. The mixture was cooled in ice bath while stirring and blowing nitrogen, and 3 mL dichloromethane solution of intermediate 025-3 (0.50 g) prepared by the previous step was added dropwise. After addition, the mixture was reacted for 2 h at the room temperature, and purified by column chromatography after completing the reaction to obtain 165 mg compound A-025. ¹H NMR (400 MHz, DMSO-*d*₆) δ 8.16 (d, *J =* 7.8 Hz, 1H), 7.95 (d, *J =* 9.6 Hz, 1H), 6.62 (s, 1H), 4.99 (s, 2H), 4.30 - 4.23 (m, 2H), 3.59 - 3.52 (m, 2H), 3.42 (s, 3H), 3.26 (s, 3H). LCMS (ESI) [M + H]⁺ =483.05, Found =482.61.

### Example 6: Preparation of compound A-149

### Step 1: Preparation of intermediate 149-1

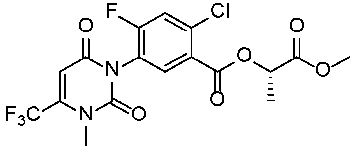

In a reaction flask, was placed 324.4 mg methyl S-(-)-lactate, which was cooled in ice bath while stirring and blowing nitrogen, and intermediate 001-8 (1 g) as described in example 1 was added dropwise, followed by 394 mg triethylamine was added dropwise. After addition, the mixture was reacted for 2 h at the room temperature, and purified by column chromatography after completing the reaction to obtain 923 mg intermediate 149-1.

### Step 2: Preparation of intermediate 149-2

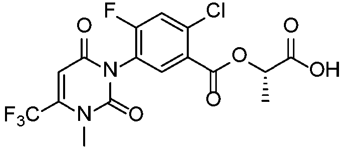

In a reaction flask, were placed 923 mg intermediate 149-1, 6.46 g hydrochloric acid (36 wt%) and 6.46 g acetic acid. The reaction solution was refluxed for 40min, and spin-dried to obtain 900 mg intermediate 149-2.

### Step 3: Preparation of intermediate 149-3

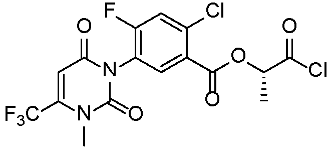

In a reaction flask, were placed 900 mg intermediate 149-2, 366.49 mg thionyl chloride, 2 drops DMF and 4.5 g 1,2-dichloroethane. The reaction solution was refluxed and reacted for 3 h, and spin-dried to obtain 800 mg intermediate 149-3.

### Step 4: Preparation of compound A-149

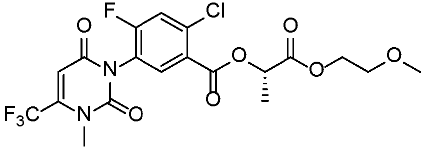

In a reaction flask, were placed 0.6 g intermediate 149-3 obtained in previous step, 0.120 g ethylene glycol monomethyl ether, 10 g dichloromethane and 0.2 g triethylamine while blowing nitrogen and stirring at room temperature for 1 h to finish the reaction. After completing the reaction, the reaction solution was purified by column chromatography to obtain 0.40 g compound A-149. ¹H NMR (400 MHz, CDCl₃) δ 7.97 (dd, *J =* 7.7, 2.0 Hz, 1H), 7.40 (d, *J* = 9.2 Hz, 1H), 6.37 (s, 1H), 5.36 (q, *J =* 7.0 Hz, 1H), 4.32 (s, 2H), 3.60 (t, *J =* 4.6 Hz, 2H), 3.57 (s, 3H), 3.36 (s, 3H), 1.62 (d, *J =* 7.0 Hz, 3H). LCMS (ESI) [M + H]⁺=497.07, Found =497.34.

### Example 7: Preparation of compound A-273

### Step 1: Preparation of compound A-249

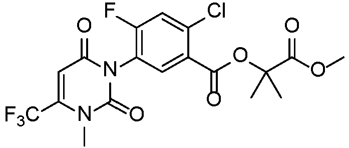

In a reaction flask, were placed 2.36 g methyl 2-hydroxyisobutyrate, 1.91 g DMAP and 50 g dichloromethane while blowing nitrogen and stirring at the room temperature, and 5 g intermediate 001-8 as described in example 1 was added dropwise within 20 min. After addition, the mixture was stirred for 1 h at room temperature, and purified by column chromatography after completing the reaction to obtain 3.57 g compound A-249.

### Step 2: Preparation of intermediate 273-2

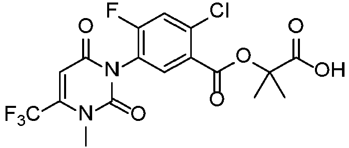

In a reaction flask, were placed 3.57 g intermediate 273-1, 20 g hydrochloric acid (36 wt%) and 20 g acetic acid. The reaction solution was stirred for 2 h at 120 °C, and poured into 100 mL ice water after completing the reaction, and extracted with EA. The organic phase was spin-dried to obtain 2.78 g intermediate 273-2.

### Step 3: Preparation of compound 273-3

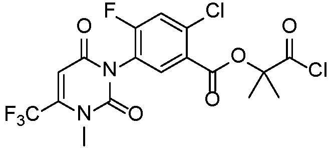

In a reaction flask, were placed 2.78 g intermediate 273-2, 1.1 g thionyl chloride, 30 g 1,2-dichloroethane and 2 drops DMF. The mixture was stirred and refluxed at 90 °C, and spin-dried after completing the reaction in one hour to obtain 3.2 g intermediate 273-3.

### Step 4: Preparation of compound A-273

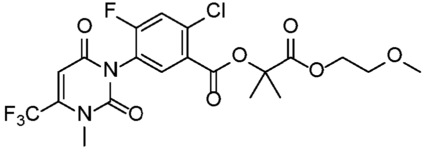

In a reaction flask, were placed 0.3 g intermediate 273-3, 0.054 g ethylene glycol monomethyl ether, 10 g dichloromethane and 0.089 g triethylamine while blowing nitrogen and stirring at room temperature. The mixture was added with 5 mL water while stirring after completing the reaction, and the organic phase was obtained by separating the liquid. The organic phase was dried, distilled under reduced pressure to remove excess solvent, and purified by column chromatography to obtain 120 mg white solid which was compound A-273. ¹H NMR (400 MHz, CDCl₃) δ 7.86 (d, *J =* 7.7 Hz, 1H), 7.38 (d, *J* = 9.2 Hz, 1H), 6.38 (s, 1H), 4.50 - 4.18 (m, 2H), 3.59 (s, 2H), 3.57 (s, 3H), 3.32 (s, 3H), 1.69 (s, 6H). LCMS (ESI) [M + H]⁺ = 511.08, Found =511.12.

### Example 8: Preparation of compound A-278

### Step 1: Preparation of compound A-278

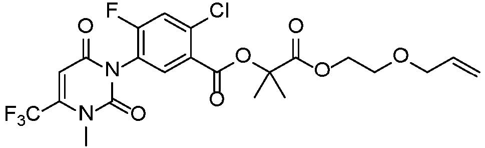

In a reaction flask, were placed 1.04 g 2-(allyloxy)ethanol, 1.24 g DMAP and 30 g dichloromethane.

The mixture was cooled in ice bath while stirring and blowing nitrogen, and 20 mL dichloromethane solution of intermediate 273-3 (3.2 g) as described in example 7 was added dropwise. After addition, the mixture was reacted for 1 h at room temperature, and purified by column chromatography after completing the reaction to obtain 600 mg compound A-278. ¹H NMR (400 MHz, DMSO-*d*₆) δ 8.07 (d, *J* = 7.8 Hz, 1H), 7.92 (d, *J = 9.6* Hz, 1H), 6.63 (s, 1H), 5.90 - 5.73 (m, 1H), 5.25 - 5.08 (m, 2H), 4.27 - 4.19 (m, 2H), 3.91 (dt, *J =* 5.3, 1.6 Hz, 2H), 3.59 - 3.55 (m, 2H), 3.42 (d, *J =* 1.3 Hz, 3H), 1.63 (s, 6H). LCMS (ESI) [M + H]⁺ =537.10, Found =536.98.

### Example 9: Preparation of a composition preparation

i) Water-soluble concentrate (SL, LS): 10-60 wt% of the composition of the present invention and 5-15 wt% of a wetting agent (e.g. alcohol alkoxylate) were dissolved in water and/or water-soluble solvent (e.g. alcohol) which was added to 100 wt%. The active ingredient dissolved when being diluted with water.
ii) Dispersible concentrate (DC): 5-25 wt% of the composition of the present invention and 1-10 wt% of dispersant (e.g. polyvinylpyrrolidone) were dissolved in an organic solvent (e.g. cyclohexanone) which was added to 100 wt%. The dispersion was obtained by dilution with water.
iii) Emulsible concentrate (EC): 15-70 wt% of the composition of the present invention and 5-10 wt% of emulsifier (e.g. calcium dodecyl benzene sulfonate and castor oil ethoxylate) were dissolved in a water-insoluble organic solvent (e.g. aromatic hydrocarbon) which was added to 100 wt%. The emulsion was obtained by dilution with water.
iv) Emulsion (EW, EO, ES): 5-40 wt% of the composition of the present invention and 1-10 wt% of emulsifier (e.g. calcium dodecyl benzene sulfonate and castor oil ethoxylate) were dissolved in 20-40 wt% of water-insoluble organic solvent (e.g. aromatic hydrocarbon). The mixture was introduced into water which was added to 100 wt% by an emulsifying machine to prepare homogeneous emulsion. The emulsion was obtained by dilution with water.
v) Suspension (SC, OD, FS): 20-60 wt% of the composition of the present invention was added with 2-10 wt% dispersant and wetting agent (e.g. sodium lignosulfonate and alcohol ethoxylate), 0.1-2 wt% thickener (e.g. xanthan gum) and water to 100 wt%, which was crushed in a stirred ball mill to obtain a fine crushed active substance suspension. A stable suspension of the active substance was obtained by dilution with water. Adhesive (e.g. polyvinyl alcohol) of up to 40 wt % were added to FS-type composition.
vi) Water-dispersible granule and water-soluble granule (WG, SG): 50-80 wt% of the composition of the present invention was added with dispersant and wetting agent (e.g. sodium lignosulfonate and alcohol ethoxylate) to 100 wt%, which was grinded finely to produce water-dispersible granule or water-soluble granule with an industrial device (e.g. extruder, spray tower, fluidized bed). A stable active substance dispersion or solution was obtained by dilution with water.
vii) Water-dispersible powder and water-soluble powder (WP, SP, WS): 50-80 wt% of the composition of the present invention was added with 1-5 wt% dispersant (e.g. sodium lignosulfonate), 1-3 wt.% wetting agent (e.g. alcohol ethoxylate) and a solid carrier (e.g. silica gel) to 100 wt.%, which was ground in a rotor-stator mill. A stable active substance dispersion or solution was obtained by dilution with water.
viii) Gel (GW, GF): 5-25 wt% of the composition of the present invention was added with 3-10 wt% of dispersant (e.g. sodium lignosulfonate), 1-5 wt% of thickener (e.g. carboxymethylcellulose) and water to 100 wt%, which was ground in a stirred ball mill to obtain a fine suspension of the active substance. A stable suspension of active substance was obtained by dilution with water.
iv) Microemulsion (ME): 5-20 wt% of the composition of the present invention was added with 5-30 wt% organic solvent blend (e.g. fatty acid dimethylamide and cyclohexanone), 10-25 wt% surfactant blend (e.g. alcohol ethoxylate and arylphenol ethoxylate), and water to 100 wt%. The mixture was stirred for 1 h to spontaneously produce a thermodynamically stable microemulsion.
iv) Microcapsule (CS): the oil phase of 5-50 wt% of the composition of the present invention, 0-40 wt% of water-insoluble organic solvents (e.g. aromatic hydrocarbon), and 2-15 wt% of acrylic monomer (e.g. methyl methacrylate, methacrylic acid, and di-or tri-acrylate) was dispersed in an aqueous solution of protective colloid (e.g. polyvinyl alcohol). The free radical polymerization initiated by free radical initiator results in the formation of poly (methyl) acrylate microcapsule. Alternatively, the oil phase of 5-50 wt% of compound I the present invention, 0-40 wt% of water-insoluble organic solvent (e.g. aromatic hydrocarbon) and isocyanate monomer (e.g. diphenylmethane-4,4'-diisocyanate) was dispersed in an aqueous solution of protective colloid (e.g. polyvinyl alcohol). The addition of polyamines (e.g. hexamethylene diamine) led to the formation of polyurea microcapsule. The monomer amount is 1-10 wt%. Wt% involved the entire CS composition. ix) Dispensable powder (DP, DS): 1-10 wt% of the composition of the present invention was finely ground and fully mixed with a solid carrier (e.g. finely crushed kaolin) which was added to 100 wt%.
x) Granule (GR, FG): 0.5-30 wt% of the composition of the present invention was finely ground and mixed with a solid carrier (e.g. silicate) which was added to 100 wt%. Granulation is achieved by extrusion, spray drying or fluidized bed.
xi) Ultra-low volume liquid (UL): 1-50 wt% of the composition of the present invention was dissolved in an organic solvent (e.g. aromatic hydrocarbon) which was added to 100 wt%.

The above preparation i)-xi) can comprise other additives optionally, such as 0.1-1 wt% fungicide, 5-15 wt% antifreeze, 0.1-1 wt% defoamer and 0.1-1 wt% colorant.

### Example 10: biological activity determination

The active ingredients A and B are respectively screened and an indoor combined effect of the composition on weeds was evaluated by an indoor test and a field test, to determine a combined effect of the composition on weeds.

### 1. Experimental method

Quantitative seeds of Poaceae weeds (*echinochloa crusgalli*, *digitaria sanguinalis*, *setaria viridis*, *eleusine indica*, *avena fatua*, *alopecurus japonicus*, *alopecurus aequalis*, *lolium multiflorum*) and broadleaf weeds (*eclipta prostrata*, *rorippa dubia*, *abutilon theophrasti*, *conyza canadensis*, *veronica persica*, *myosoton aquaticum*, *polygonum lapathifolium*, *galium aparine var. tenerum*, *alisma plantago-aquatica*, *geranium carolinianum*) were sown in paper cup containing nutrient soil with a diameter of 7 cm, covered with soil for 1 cm after sowing. After pressing and watering, they were cultured in a greenhouse according to a conventional method. Poaceae weeds grew to 3~4 leaf stage, broad-leaved weeds grew to 4~6 leaf stage, spraying treatment to both stem and leaf was performed; and pre-seedling soil spray treatment was performed 24 hours after sowing. Following an experimental design dosage, a WP-2000 walking spray tower (spindle speed 96 mm/r, spray height 200 mm, effective spray amplitude of nozzle 350 mm, nozzle flow rate 390 mL/min, nozzle walking speed 210 mm/s) was used for the spray treatment. The experiment was repeated three times. After being treated, a sample material is placed in an operation hall, and after the drug solution is naturally dried out, the sample material is placed in a greenhouse and managed according to the conventional method. A reaction of weeds to the drug agent is observed and recorded, and a regularly visual check of a control effect of the drug agent on weeds was performed after the treatment.

### 2. Experimental Design

### (1) Experimental agents

All of original drugs were using acetone as a solvent, being diluted with 0.1 wt% emulsifier of Tween-80 aqueous solution, and being diluted right before using.

### (2) Dosage setting

When determining a ratio or content of the active ingredient A and the active ingredient B, it should be measured from characteristics of action of both agents and virulence thereof, as well as the main purpose of use of a formula shall also be considered in. In the present study, on a basis of previous pre-test, the dosages of the active ingredient A and the active ingredient B were set, when being used alone and mixed, as shown in the following table. Water containing no agent, but containing the same solvent and emulsifier was used as a blank control.

### (3) Experimental repeating

Repeat 4 times for each treatment, 3 pots per treatment each time and 30 weed seeds were sown for each pot.

### (4) Survey methods

Adopting an absolute number survey method, using a blade to cut the whole seedling of surviving weed along soil surface, and weighting the fresh weight of the weed with an analytical balance. For weeds that had died, counting the fresh weight as zero.

Survey period and frequency: 21 days after the treatment, total 1 investigation.

### (5) Data statistical analysis

The theoretical fresh weight inhibition rate (E0-Expected Efficacy=X+YX*Y/100) of each treatment combination was calculated by Gowing method, and then compared with the measured inhibition rate (E-Observed Efficacy) to evaluate the combined effect type of both on weeds. When E-EO value was greater than 10%, it was counted as a synergistic effect, less than -10%, it was counted as an antagonistic effect, and between -10%~10%, it was counted as an additive effect. According to a plurality of factors including an actual control efficacy, a herbicide characteristic, a formula balance and other factors to determine the best ratio. In the formula, X is the fresh weight inhibition rate of the ingredient A at a dosage of P; Y is the fresh weight inhibition rate of the active ingredient B at a dosage of Q.

The statistical results are shown in Table 1 to Table 44 below, except for those marked clearly as "pre-emergence treatment", all others are "post-emergence treatment". The dosages of A single agent and B single agent in the following table are the same as those in the composition of A and B. For example, in Table 1, when the dosage of A + B is 25 + 12.5 (g/ha) and the ratio of A to B is 2:1, the dosage of A single agent is 25 g/ha, and the dosage of B single agent is 12.5 g/ha.

**Table 1 Evaluation of indoor virulence and combined effect of compound A and nicosulfuron on digitaria sanguinalis**

| Name of the agents | A+B dosage (g a.i./ha) | A:B | Fresh weight inhibition rate % | | | E0 | E-E0 |
|---|---|---|---|---|---|---|---|
| | | | A single agent | B single agent | A+B, E | | |
| A: A-250 | 25+12.5 | 2:1 | | | | | |
| | 20+20 | 1:1 | | | | | |
| B: nicosulfuron | 12.5+25 | 1:2 | | | | | |
| | 10+30 | 1:3 | | | | | |
| A: A-273 | 25+12.5 | 2:1 | 62.48 | 10.24 | 81.5 | 66.32 | 15.18 |
| | 20+20 | 1:1 | 60.47 | 23.38 | 80.00 | 69.71 | 10.29 |
| B: nicosulfuron | 12.5+25 | 1:2 | 55.30 | 34.90 | 84.33 | 70.90 | 13.43 |
| | 10+30 | 1:3 | 45.50 | 39.13 | 80.36 | 66.83 | 13.54 |

**Table 2 Evaluation of indoor virulence and combined effect of compound A and penoxsulam on echinochloa crusgalli**

| Name of the agents | A+B dosage (g a.i./ha) | A:B | Fresh weight inhibition rate % | | | E0 | E-E0 |
|---|---|---|---|---|---|---|---|
| | | | A single agent | B single agent | A+B, E | | |
| A: A-250 | 15+5 | 3:1 | 26.41 | 7.31 | 58.64 | 31.79 | 26.85 |
| B: penoxsulam | 20+10 | 2:1 | 30.73 | 18.27 | 81.56 | 43.39 | 38.17 |
| A: A-273 | 15+5 | 3:1 | | | | | |
| B: penoxsulam | 20+10 | 2:1 | | | | | |

**Table 3 Evaluation of indoor virulence and combined effect of compound A and imazamox-sodium on echinochloa crusgalli**

| Name of the agents | A+B dosage (g a.i./ha) | A:B | Fresh weight inhibition rate % | | | E0 | E-E0 |
|---|---|---|---|---|---|---|---|
| | | | A single agent | B single agent | A+B, E | | |
| A: A-250 | 25+15 | 1:0.6 | 31.56 | 2.52 | 63.93 | 33.28 | 30.65 |
| B:imazamox-sodium | 20+20 | 1:1 | 30.73 | 13.41 | 56.63 | 40.02 | 16.61 |
| A: A-273 | 25+15 | 1:0.6 | | | | | |
| B:imazamox-sodium | 20+20 | 1:1 | | | | | |

**Table 4 Evaluation of indoor virulence and combined effect of compound A and bispyribac-sodium on echinochloa crusgalli**

| Name of the agents | A+B dosage (g a.i./ha) | A:B | Fresh weight inhibition rate % | | | E0 | E-E0 |
|---|---|---|---|---|---|---|---|
| | | | A single agent | B single agent | A+B, E | | |
| A: A-250 | 25+25 | 1:1 | 31.56 | 30.15 | 75.93 | 52.19 | 23.74 |
| B: bispyribac-sodium | 20+10 | 2:1 | 30.73 | 21.85 | 61.26 | 45.87 | 15.39 |
| | 15+5 | 3:1 | 26.41 | 14.30 | 53.19 | 36.93 | 16.25 |
| A: A-273 | 25+25 | 1:1 | | | | | |
| B: bispyribac-sodium | 20+10 | 2:1 | | | | | |
| | 15+5 | 3:1 | | | | | |

**Table 5 Evaluation of indoor virulence and combined effect of compound A and clethodim on alopecurus japonicus**

| Name of the agents | A+B dosage (g a.i./ha) | A:B | Fresh weight inhibition rate % | | | E0 | E-E0 |
|---|---|---|---|---|---|---|---|
| | | | A single agent | B Single agent | A+B, E | | |
| A: A-250 | 25+25 | 1:1 | | | | | |
| B: clethodim | 20+40 | 1:2 | | | | | |
| | 15+60 | 1:4 | | | | | |
| A: A-273 | 25+25 | 1:1 | 72.42 | 7.57 | 86.91 | 74.49 | 12.42 |
| B: clethodim | 20+40 | 1:2 | 61.55 | 34.82 | 91.36 | 74.94 | 16.42 |
| | 15+60 | 1:4 | 44.57 | 42.99 | 87.99 | 68.40 | 19.59 |

**Table 6 Evaluation of indoor virulence and combined effect of compound A and propanil on setaria viridis**

| Name of the agents | A+B dosage (g a.i./ha) | A:B | Fresh weight inhibition rate % | | | E0 | E-E0 |
|---|---|---|---|---|---|---|---|
| | | | A single agent | B single agent | A+B, E | | |
| A: A-250 | 20+500 | 1:25 | 78.77 | 48.06 | 100 | 88.97 | 11.03 |
| B: propanil | 15+750 | 1:50 | 65.7 | 58.06 | 99.37 | 85.61 | 13.75 |
| | 10+1000 | 1:100 | 52.54 | 69.11 | 100 | 85.34 | 14.66 |
| A: A-273 | 20+500 | 1:25 | | | | | |
| B: propanil | 15+750 | 1:50 | | | | | |
| | 10+1000 | 1:100 | | | | | |

**Table 7 Evaluation of indoor virulence and combined effect of compound A and pinoxaden on alopecurus japonicus**

| Name of the agents | A+B dosage (g a.i./ha) | A: B | Fresh weight inhibition rate % | | | E0 | E-EO |
|---|---|---|---|---|---|---|---|
| | | | A single agent | B single agent | A+B, E | | |
| A: A-250 | 25+15 | 1:0.6 | 55.39 | 38.8 | 87.14 | *72.70* | 14.44 |
| B: pinoxaden | 20+20 | 1:1 | 49.17 | 42.32 | 95.44 | 70.68 | 24.76 |
| | 15+25 | 0.6:1 | 39 | 58.71 | 97.51 | 74.81 | 22.70 |
| A: A-273 | 25+15 | 1:0.6 | | | | | |
| B: pinoxaden | 20+20 | 1:1 | | | | | |
| | 15+25 | 0.6:1 | | | | | |

**Table 8 Evaluation of indoor virulence and combined effect of compound A and atrazine on setaria viridis**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 20+600 | 1:30 | 78.77 | 26.07 | 100 | 84.3 | 15.7 |
| B: atrazine | 15+750 | 1:50 | 65.7 | 32.97 | 100 | 77 | 23 |
| | 10+800 | 1:80 | 52.54 | 41.63 | 100 | 72.3 | 27.7 |
| A: A-273 | 20+600 | 1:30 | | | | | |
| B: atrazine | 15+750 | 1:50 | | | | | |
| | 10+800 | 1:80 | | | | | |

**Table 9 Evaluation of indoor virulence and combined effect of compound A and bentazone-sodium on veronica persica**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 20+300 | 1:15 | 82.86 | 30.46 | 100.00 | 88.08 | 11.92 |
| B: bentazone-sodium | 15+450 | 1:30 | 70.47 | 41.88 | 100.00 | 82.84 | 17.16 |
| | 10+525 | 1:52.5 | 68.79 | 53.29 | 99.20 | 85.42 | 13.78 |
| A: A-273 | 20+300 | 1:15 | | | | | |
| B: bentazone-sodium | 15+450 | 1:30 | | | | | |
| | 10+525 | 1:52.5 | | | | | |

**Table 10 Evaluation of indoor virulence and combined effect of compound A and isoproturon on setaria viridis**

| Name of the agents | A+B dosage (g a.i./ha) | A: B | Fresh weight inhibition rate % | | | E0 | E-EO |
|---|---|---|---|---|---|---|---|
| | | | A single agent | B single agent | A+B, E | | |
| A: A-250 | 20+400 | 1:20 | 78.77 | 22.19 | 100 | 83.48 | 16.52 |
| B: isoproturon | 15+495 | 1:33 | 65.7 | 23.26 | 97.99 | 73.68 | 24.32 |
| | 10+600 | 1:60 | 52.54 | 33.26 | 91.84 | 68.33 | 23.52 |
| A: A-273 | 20+400 | 1:20 | | | | | |
| B: isoproturon | 15+495 | 1:33 | | | | | |
| | 10+600 | 1:60 | | | | | |

**Table 11 Evaluation of indoor virulence and combined effect of compound A and flumioxazin on eclipta prostrata**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 10+30 | 1:3 | | | | | |
| B: flumioxazin | | | | | | | |
| A: A-273 | 2+30 | 1:15 | 28.12 | 54.24 | 78.62 | 67.11 | 11.51 |
| | 4+20 | 1:5 | 38.50 | 45.24 | 84.30 | 66.32 | 17.98 |
| B: flumioxazin | 8+16 | 1:2 | 46.05 | 39.68 | 91.97 | 67.46 | 24.51 |
| | 16+8 | 2:1 | 52.89 | 24.97 | 95.18 | 64.66 | 30.52 |

**Table 12 Evaluation of indoor virulence and combined effect of compound A and fluoroglycofen on eclipta prostrata**

| Name of the agents | A+B dosage (g a.i./ha) | A:B | Fresh weight inhibition rate% | | | E0 | E-EO |
|---|---|---|---|---|---|---|---|
| | | | A single agent | B single agent | A+B, E | | |
| A: A-250 | 4+20 | 1:5 | | | | | |
| B: fluoroglycofen-ethyl | 8+16 | 1:2 | | | | | |
| | 16+8 | 2:1 | | | | | |
| A: A-273 | 4+20 | 1:5 | 38.50 | 42.03 | 88.50 | 64.35 | 24.16 |
| B: fluoroglycofen-ethyl | 8+16 | 1:2 | 46.05 | 27.44 | 89.49 | 60.86 | 28.64 |
| | 16+8 | 2:1 | 52.89 | 11.99 | 88.76 | 58.54 | 30.22 |

**Table 13 Evaluation of indoor virulence and combined effect of compound A mixed with mesotrione on digitaria sanguinalis**

| Name of the agents | A+B dosage (g a.i./ha) | A: B | Fresh weight inhibition rate % | | | E0 | E-EO |
|---|---|---|---|---|---|---|---|
| | | | A single agent | B single agent | A+B, E | | |
| A: A-250 | 30+90 | 1:3 | | | | | |
| B: mesotrione | 25+105 | 1:4.2 | | | | | |
| | 20+120 | 1:6 | | | | | |
| A: A-273 | 30+90 | 1:3 | 63.97 | 45.39 | 90.62 | 80.32 | 10.3 |
| B: mesotrione | 25+105 | 1:4.2 | 62.48 | 56.62 | 96.15 | 83.72 | 12.43 |
| | 20+120 | 1:6 | 60.47 | 58.12 | 95.64 | 83.45 | 12.2 |

**Table 14 Evaluation of indoor virulence and combined effect of compound A mixed with flusulfinam on digitaria sanguinalis**

| Name of the agents | A+B dosage (g a.i./ha) | A:B | Fresh weight suppression rate % | | | E0 | E-EO |
|---|---|---|---|---|---|---|---|
| | | | A single agent | B single agent | A+B, E | | |
| A: A-250 | 30+15 | 2:1 | | | | | |
| | 20+30 | 1:1.5 | | | | | |
| B: flusulfinam | 15+37.5 | 1:2.5 | | | | | |
| | 10+45 | 1:4.5 | | | | | |
| A: A-273 | 30+15 | 2:1 | 65.40 | 34.84 | 87.54 | 77.45 | 10.08 |
| | 20+30 | 1:1.5 | 60.47 | 36.52 | 95.04 | 74.90 | 20.14 |
| B: flusulfinam | 15+37.5 | 1:2.5 | 59.30 | 40.37 | 95.16 | 75.73 | 19.43 |
| | 10+45 | 1:4.5 | 31.16 | 50.75 | 77.23 | 66.10 | 11.13 |

**Table 15 Evaluation of indoor virulence and combined effect of compound A and topramezone on digitaria sanguinalis**

| Name of the agents | A+B dosage (g a.i./ha) | A: B | Fresh weight inhibition rate % | | | E0 | E-EO |
|---|---|---|---|---|---|---|---|
| | | | A single agent | B single agent | A+B, E | | |
| A: A-250 | 25+7.5 | 3.3:1 | | | | | |
| B: topramezone | 20+10 | 2:1 | | | | | |
| | 15+12.5 | 1.2:1 | | | | | |
| A: A-273 | 25+7.5 | 3.3:1 | 62.48 | 3.85 | 87.38 | 63.92 | 23.46 |
| B: topramezone | 20+10 | 2:1 | 60.47 | 17.09 | 85.21 | 67.23 | 17.98 |
| | 15+12.5 | 1.2:1 | 59.3 | 19.6 | 87.24 | 67.28 | 19.96 |

**Table 16 Evaluation of indoor toxicity and combined effect of compound A and isoxaflutole on digitaria sanguinalis**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 30+40 | 1:1.3 | | | | | |
| | 25+50 | 1:2 | | | | | |
| B: isoxaflutole | 20+60 | 1:3.6 | | | | | |
| | 10+70 | 1:7 | | | | | |
| A: A-273 | 30+40 | 1:1.3 | 60.30 | 56.12 | 100.00 | 82.58 | 17.42 |
| | 25+50 | 1:2 | 58.81 | 71.94 | 98.51 | 88.44 | 10.07 |
| B: isoxaflutole | 20+60 | 1:3.6 | 45.07 | 73.13 | 100.00 | 85.24 | 14.76 |
| | 10+70 | 1:7 | 9.55 | 76.72 | 88.96 | 78.94 | 10.01 |

**Table 17 Evaluation of indoor virulence and combined effect of compound A and clomazone on echinochloa crusgalli**

| Name of the agents | A+B dosage (g a.i./ha) | A: B | Fresh weight inhibition rate % | | | E0 | E-EO |
|---|---|---|---|---|---|---|---|
| | | | A single agent | B single agent | A+B, E | | |
| A: A-250 | | | | | | | |
| B: clomazone | | | | | | | |
| | | | | | | | |
| A: A-273 | 25+12.5 | 2:1 | 19.41 | 8.42 | 53.30 | 26.20 | 27.10 |
| B: clomazone | 20+20 | 1:1 | 21.43 | 7.51 | 54.03 | 27.33 | 26.70 |
| | 15+45 | 1:3 | 30.04 | 10.92 | 49.25 | 37.67 | 11.58 |

**Table 18 Evaluation of indoor virulence and combined effect of compound A and glyphosate-isopropylammonium on eleusine indica**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-249 | 5+500 | 1:100 | | | | | |
| B: glyphosate-isopropylammonium | 10+350 | 1:35 | | | | | |
| | 15+240 | 1:16 | | | | | |
| | 20+160 | 1:8 | | | | | |
| A: A-250 | 5+500 | 1:100 | | | | | |
| B: glyphosate-isopropylammonium | 10+350 | 1:35 | | | | | |
| | 15+240 | 1:16 | | | | | |
| | 20+160 | 1:8 | | | | | |
| A: A-273 | 5+500 | 1:100 | 34.65 | 45.72 | 92.90 | 64.53 | 28.37 |
| B: glyphosate-isopropylammonium | 10+350 | 1:35 | 43.34 | 35.52 | 97.22 | 63.47 | 33.76 |
| | 15+240 | 1:16 | 55.00 | 32.37 | 91.99 | 69.5 | 22.43 |
| | 20+160 | 1:8 | 68.03 | 27.36 | 94.99 | 76.78 | 18.22 |

**Table 19 Evaluation of indoor virulence and combined effect of compound A and glufosinate-ammonium on lolium multiflorum**

| Name of the agents | A+B dosage (g a.i./ha) | A:B | Fresh weight inhibition rate rate % | | A+B, E | E0 | E-EO |
|---|---|---|---|---|---|---|---|
| | | | A single agent | B single agent | | | |
| A: A-249 | 5+400 | 1:80 | | | | | |
| B: glufosinate-ammonium | 10+300 | 1:30 | | | | | |
| | 15+210 | 1:14 | | | | | |
| | 20+120 | 1:6 | | | | | |
| A: A-250 | 5+400 | 1:80 | | | | | |
| B: glufosinate-ammonium | 10+300 | 1:30 | | | | | |
| | 15+210 | 1:14 | | | | | |
| | 20+120 | 1:6 | | | | | |
| A: A-273 | 5+400 | 1:80 | 22.25 | 54.64 | 79.67 | 64.73 | 14.93 |
| B: glufosinate-ammonium | 10+300 | 1:30 | 50.48 | 27.99 | 89.00 | 64.34 | 24.66 |
| | 15+210 | 1:14 | 55.50 | 12.20 | 92.11 | 60.93 | 31.17 |
| | 20+120 | 1:6 | 60.05 | 2.63 | 95.93 | 61.10 | 34.83 |

**Table 20 Evaluation of indoor virulence and combined effect of compound A and glufosinate-P-ammonium on lolium multiflorum**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-249 | 20+200 | 1:10 | | | | | |
| B: glufosinate-P-ammonium | 15+300 | 1:20 | | | | | |
| | 10+350 | 1:35 | | | | | |
| | 5+400 | 1:80 | | | | | |
| A: A-250 | 20+200 | 1:10 | | | | | |
| B: glufosinate-P-ammonium | 15+300 | 1:20 | | | | | |
| | 10+350 | 1:35 | | | | | |
| | 5+400 | 1:80 | | | | | |
| A: A-273 | 20+200 | 1:10 | 60.05 | 8.61 | 100.00 | 63.49 | 36.51 |
| B: glufosinate-P-ammonium | 15+300 | 1:20 | 55.50 | 74.40 | 100.00 | 88.61 | 11.39 |
| | 10+350 | 1:35 | 50.48 | 78.23 | 100.00 | 89.22 | 10.78 |
| | 5+400 | 1:80 | 22.25 | 83.04 | 98.58 | 86.81 | 11.77 |

**Table 21 Evaluation of indoor virulence and combined effect of compound A and pendimethalin on eleusine indica (pre-emergence treatment)**

| Name of the agents | A+B dosage (g a.i./ha) | A : B | Fresh weight inhibition rate % | | | E0 | E-EO |
|---|---|---|---|---|---|---|---|
| | | | A single agent | B single agent | A+B, E | | |
| A: A-250 | 30+300 | 1:10 | 90.86 | 36.92 | 100.00 | 60.05 | 39.95 |
| B: pendimethalin | 25+375 | 1:15 | 69.44 | 54.44 | 96.96 | 82.83 | 14.13 |
| A: A-273 | 30+300 | 1:10 | | | | | |
| B: pendimethalin | 25+375 | 1:15 | | | | | |

**Table 22 Evaluation of indoor virulence and combined effect of compound A and anilofos on echinochloa crusgalli**

| Name of the agents | A+B dosage (g a.i./ha) | A:B | Fresh weight suppression rate % | | | E0 | E-EO |
|---|---|---|---|---|---|---|---|
| | | | A single dose | B single dose | A+B, E | | |
| A: A-250 | 25+60 | 1:2.4 | 31.56 | 37.41 | 96.15 | 57.17 | 38.99 |
| | 20+80 | 1:4 | 30.73 | 41.96 | 79.72 | 59.79 | 19.93 |
| B: anilofos | 15+105 | 1:7 | 26.41 | 46.85 | 89.16 | 60.89 | 28.27 |
| | 10+120 | 1:12 | 17.77 | 64.69 | 89.51 | 70.96 | 18.55 |
| A: A-273 | 25+60 | 1:2.4 | | | | | |
| | 20+80 | 1:4 | | | | | |
| B: anilofos | 15+105 | 1:7 | | | | | |
| | 10+120 | 1:12 | | | | | |

**Table 23 Evaluation of indoor virulence and combined effect of compound A and 2,4-D-etexyl on veronica persica**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 5+350 | 1:70 | 62.6 | 50.28 | 97.5 | 81.4 | 16.1 |
| B: 2,4-D-etexyl | 10+275 | 1:27.5 | 68.79 | 40.62 | 100 | 81.47 | 18.53 |
| | 15+195 | 1:13 | 70.47 | 30.42 | 100 | 79.45 | 20.55 |
| A: A-273 | 5+350 | 1:70 | | | | | |
| B: 2,4-D-etexyl | 10+275 | 1:27.5 | | | | | |
| | 15+195 | 1:13 | | | | | |

**Table 24 Evaluation of indoor virulence and combined effect of compound A and dicambadimethylammonium on veronica persica**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 20+40 | 1:2 | 82.86 | 11.68 | 100 | 84.86 | 15.14 |
| B: dicamba-dimethylammonium | 15+60 | 1:4 | 70.47 | 25.89 | 100 | 78.11 | 21.89 |
| | 10+80 | 1:8 | 68.79 | 37.82 | 100 | 80.6 | 19.4 |
| | 5+100 | 1:25 | 62.6 | 38.58 | 95.6 | 77.03 | 18.57 |
| A: A-273 | 20+40 | 1:2 | | | | | |
| B: dicamba-dimethylammonium | 15+60 | 1:4 | | | | | |
| | 10+80 | 1:8 | | | | | |
| | 5+100 | 1:25 | | | | | |

**Table 25 Evaluation of indoor virulence and combined effect of compound A and haloxyfop-P-methyl on alopecurus japonicus**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 30+15 | 2:1 | | | | | |
| B: haloxyfop-P-methyl | 25+25 | 1:1 | | | | | |
| | 20+40 | 1:2 | | | | | |
| | 15+60 | 1:4 | | | | | |
| A: A-273 | 30+15 | 2:1 | 86.91 | 7.80 | 100.00 | 87.93 | 12.07 |
| B: haloxyfop-P-methyl | 25+25 | 1:1 | 72.42 | 15.29 | 100.00 | 76.64 | 23.36 |
| | 20+40 | 1:2 | 61.55 | 15.32 | 100.00 | 67.44 | 32.56 |
| | 15+60 | 1:4 | 44.57 | 27.52 | 93.04 | 59.82 | 33.22 |

**Table 26 Evaluation of indoor virulence and combined effect of compound A and picloram on veronica persica**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 15+450 | 1:30 | 70.47 | 42.89 | 100 | 83.14 | 16.86 |
| B: picloram-potassium | 10+525 | 1:52.5 | 68.79 | 47.46 | 100 | 83.60 | 16.40 |
| | 5+600 | 1:120 | 62.6 | 50.25 | 100 | 81.39 | 18.61 |
| | 2+600 | 1:300 | 24.6 | 50.25 | 78.9 | 62.49 | 16.41 |
| A: A-273 | 15+450 | 1:30 | | | | | |
| B: picloram-potassium | 10+525 | 1:52.5 | | | | | |
| | 5+600 | 1:120 | | | | | |
| | 2+600 | 1:300 | | | | | |

**Table 27 Evaluation of indoor virulence and combined effect of compound A and diquat on lolium multiflorum**

| Name of the agents | A+B dosage (g a.i./ha) | A: B | Fresh weight inhibition rate % | | | E0 | E-EO |
|---|---|---|---|---|---|---|---|
| | | | A single agent | B single agent | A+B, E | | |
| A: A-250 | 25+300 | 1:12 | | | | | |
| B: diquat | 20+400 | 1:20 | | | | | |
| | 15+495 | 1:33 | | | | | |
| A: A-273 | 25+300 | 1:12 | 77.30 | 31.27 | 100.00 | 84.40 | 15.60 |
| B: diquat | 20+400 | 1:20 | 60.05 | 36.44 | 100.00 | 74.61 | 25.39 |
| | 15+495 | 1:33 | 55.5 | 41.37 | 100.00 | 73.91 | 26.09 |

**Table 28 Evaluation of indoor virulence and combined effect of compound A and S-metolachlor on eclipta prostrata (pre-emergence treatment)**

| Name of the agent | A+B dosage (g a.i./ha) | A: B | Fresh weight inhibition rate % | | | E0 | E-EO |
|---|---|---|---|---|---|---|---|
| | | | A single agent | B single agent | A+B, E | | |
| A: A-250 | 20+300 | 1:15 | 78.12 | 45.64 | 100 | 88.11 | 11.89 |
| | 15+450 | 1:30 | 68.5 | 57.41 | 100 | 86.58 | 13.42 |
| B: S-metolachlor | 10+600 | 1:60 | 66.05 | 61.78 | 100 | 87.03 | 12.97 |
| | 5+750 | 1:150 | 52.89 | 70.72 | 100 | 86.21 | 13.79 |
| A: A-273 | 20+300 | 1:15 | | | | | |
| | 15+450 | 1:30 | | | | | |
| B: S-metolachlor | 10+600 | 1:60 | | | | | |
| | 5+750 | 1:150 | | | | | |

**Table 29 Evaluation of indoor virulence and combined effect of compound A and metamifop on digitaria sanguinalis**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 15+60 | 1:4 | 54.46 | 60.50 | 96.05 | 82.01 | 14.04 |
| | 20+40 | 1:2 | 61.09 | 58.52 | 97.24 | 83.86 | 13.38 |
| B: metamifop | 25+25 | 1:1 | 65.86 | 55.48 | 94.88 | 84.80 | 10.08 |
| | 30+15 | 2:1 | 71.11 | 49.80 | 95.83 | 85.50 | 10.33 |
| | 15+60 | 1:4 | | | | | |
| A: A-273 | 20+40 | 1:2 | | | | | |
| B: metamifop | 25+25 | 1:1 | | | | | |
| | 30+15 | 2:1 | | | | | |

**Table 30 Evaluation of indoor virulence and combined effect of compound A and saflufenacil on eleusine indica**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 10+40 | 1:4 | 43.34 | 50.09 | 88.94 | 71.72 | 17.22 |
| | 15+30 | 1:2 | 55.00 | 58.13 | 92.71 | 81.16 | 11.55 |
| B: saflufenacil | 20+20 | 1:1 | 68.03 | 50.62 | 95.96 | 84.21 | 11.75 |
| | 25+12.5 | 2:1 | 70.36 | 39.66 | 93.97 | 82.12 | 11.86 |
| | 30+7.5 | 4:1 | 75.35 | 23.20 | 93.58 | 81.07 | 12.51 |
| A: A-273 | 10+40 | 1:4 | | | | | |
| | 15+30 | 1:2 | | | | | |
| B: saflufenacil | 20+20 | 1:1 | | | | | |
| | 25+12.5 | 2:1 | | | | | |
| | 30+7.5 | 4:1 | | | | | |

**Table 31 Evaluation of indoor virulence and combined effect of compound A and oxadiargyl on digitaria sanguinalis**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 15+30 | 1:2 | 54.46 | 52.31 | 88.90 | 78.28 | 10.62 |
| B: oxadiargyl | 20+20 | 1:1 | 61.09 | 42.31 | 89.79 | 77.55 | 12.24 |
| | 25+12.5 | 2:1 | 65.86 | 38.31 | 93.26 | 78.94 | 14.32 |
| A: A-273 | 15+30 | 1:2 | | | | | |
| B: oxadiargyl | 20+20 | 1:1 | | | | | |
| | 25+12.5 | 2:1 | | | | | |

**Table 32 Evaluation of indoor virulence and combined effect of compound A and sulfentrazone on eleusine indica**

| Name of the agent | A+B dosage (g a.i./ha) | A:B | Fresh weight inhibition rate % | | | E0 | E-E0 |
|---|---|---|---|---|---|---|---|
| | | | A single agent | B single agent | A+B, E | | |
| A: A-250 B: sulfentrazone | | | | | | | |
| | | | | | | | |
| | | | | | | | |
| A: A-273 | 25+75 | 1:3 | 70.36 | 8.30 | 83.59 | 72.82 | 10.77 |
| B: sulfentrazone | 20+120 | 1:6 | 68.03 | 18.12 | 85.10 | 73.82 | 11.28 |
| | 15+150 | 1:10 | 55.00 | 23.53 | 82.58 | 65.59 | 16.99 |

**Table 33**

| Evaluation of indoor virulence and combined effect of compound A and SY-1604(CAS NO.:1949837-17-5) on *eleusine indica* | | | | | | | |
|---|---|---|---|---|---|---|---|
| Name of the agents | A+B dosage (g a.i./ha) | A:B | Fresh weight inhibition rate % | | | E0 | E-E0 |
| | | | A single agent | B single agent | A+B, E | | |
| A: A-250 | 10+40 | 1:4 | | | | | |
| | 15+30 | 1:2 | | | | | |
| B:SY-1604(CAS NO.: 1949837-17-5) | 20+20 | 1:1 | | | | | |
| | 25+12.5 | 2:1 | | | | | |
| | 30+7.5 | 4:1 | | | | | |
| A: A-273 | 10+40 | 1:4 | 43.34 | 74.06 | 97.21 | 85.30 | 11.91 |
| | 15+30 | 1:2 | 55.00 | 72.50 | 97.82 | 87.63 | 10.19 |
| B:SY-1604(CAS NO.: 1949837-17-5) | 20+20 | 1:1 | 68.03 | 64.09 | 98.70 | 88.52 | 10.18 |
| | 25+12.5 | 2:1 | 70.36 | 48.33 | 94.71 | 84.69 | 10.03 |
| | 30+7.5 | 4:1 | 75.35 | 47.10 | 97.54 | 86.96 | 10.58 |

**Table 34 Evaluation of indoor virulence and combined effect of compound A and butafenacil on lolium multiflorum**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 15+30 | 1:2 | 55.00 | 53.40 | 94.71 | 79.03 | 15.67 |
| | 20+20 | 1:1 | 68.03 | 43.60 | 92.35 | 81.97 | 10.38 |
| B: butafenacil | 25+12.5 | 2:1 | 70.36 | 36.55 | 93.45 | 81.20 | 12.26 |
| | 30+7.5 | 4:1 | 75.35 | 29.47 | 94.03 | 82.62 | 11.41 |
| A: A-273 | 15+30 | 1:2 | | | | | |
| | 20+20 | 1:1 | | | | | |
| B: butafenacil | 25+12.5 | 2:1 | | | | | |
| | 30+7.5 | 4:1 | | | | | |

**Table 35 Evaluation of indoor virulence and combined effect of compound A and epyrifenacil on eleusine indica**

| Name of the agents | A+B dosage (g a.i./ha) | A: B | Fresh weight inhibition rate % | | | E0 | E-EO |
|---|---|---|---|---|---|---|---|
| | | | A single agent | B single agent | A+B, E | | |
| A: A-250 | 10+20 | 1:2 | | | | | |
| | 15+15 | 1:1 | | | | | |
| B: epyrifenacil | 20+10 | 2:1 | | | | | |
| | 25+5 | 5:1 | | | | | |
| A: A-273 | 10+20 | 1:2 | 43.34 | 51.59 | 88.31 | 72.57 | 15.74 |
| | 15+15 | 1:1 | 55.00 | 37.91 | 86.71 | 72.06 | 14.65 |
| B: epyrifenacil | 20+10 | 2:1 | 68.03 | 32.92 | 90.34 | 78.55 | 11.79 |
| | 25+5 | 5:1 | 70.36 | 38.04 | 97.21 | 81.64 | 15.58 |

**Table 36 Evaluation of indoor virulence and combined effect of compound A and flufenoximacil on eleusine indica**

| Name of the agents | A+B dosage (g a.i./ha) | A: B | Fresh weight inhibition rate % | | | E0 | E-EO |
|---|---|---|---|---|---|---|---|
| | | | A single agent | B single agent | A+B, E | | |
| A: A-250 | 5+15 | 1:3 | | | | | |
| | 10+20 | 1:2 | | | | | |
| B: flufenoximacil | 15+15 | 1:1 | | | | | |
| | 20+10 | 2:1 | | | | | |
| | 25+5 | 5:1 | | | | | |
| A: A-273 | 5+15 | 1:3 | 34.65 | 55.99 | 95.72 | 71.24 | 24.48 |
| | 10+20 | 1:2 | 43.34 | 59.18 | 99.68 | 76.87 | 22.81 |
| B: flufenoximacil | 15+15 | 1:1 | 55.00 | 55.99 | 98.57 | 80.20 | 18.38 |
| | 20+10 | 2:1 | 68.03 | 51.91 | 99.61 | 84.63 | 14.99 |
| | 25+5 | 5:1 | 70.36 | 34.41 | 98.12 | 80.56 | 17.56 |

**Table 37 Evaluation of indoor virulence and combined effect of compound A and cyclopyranil on echinochloa crusgalli**

| Name of the agents | A+B dosage (g a.i./ha) | A: B | Fresh weight inhibition rate % | | | E0 | E-EO |
|---|---|---|---|---|---|---|---|
| | | | A single agent | B Single agent | A+B, E | | |
| A: A-250 | | | | | | | |
| | | | | | | | |
| B: cyclopyranil | | | | | | | |
| | | | | | | | |
| | 10+40 | 1:4 | 19.41 | 39.05 | 71.25 | 50.88 | 20.36 |
| A: A-273 | 15+30 | 1:2 | 21.43 | 31.50 | 76.92 | 46.18 | 30.74 |
| B: cyclopyranil | 20+20 | 1:1 | 30.04 | 25.46 | 91.58 | 47.85 | 43.73 |
| | 25+12.5 | 2:1 | 38.28 | 24.73 | 78.75 | 53.54 | 25.21 |

**Table 38 Evaluation of indoor virulence and combined effect of compound A and tiafenacil on lolium multiflorum**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 10+20 | 1:2 | 43.34 | 22.42 | 67.52 | 56.04 | 11.48 |
| B: tiafenacil | 15+15 | 1:1 | 55.00 | 15.26 | 74.17 | 61.87 | 12.30 |
| | 20+10 | 2:1 | 68.03 | 8.27 | 85.79 | 70.67 | 15.11 |
| A: A-273 | 10+20 | 1:2 | | | | | |
| B: tiafenacil | 15+15 | 1:1 | | | | | |
| | 20+10 | 2:1 | | | | | |

**Table 39 Evaluation of indoor virulence and joint effect of compound A and trifluralin on digitaria sanguinalis (pre-emergence treatment)**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 15+375 | 1:25 | 68.96 | 62.58 | 100.00 | 88.38 | 11.62 |
| B: trifluralin | 20+250 | 1:12.5 | 75.50 | 48.67 | 100.00 | 87.42 | 12.58 |
| | 25+156.25 | 1:6.25 | 85.50 | 12.50 | 100.00 | 87.31 | 12.69 |
| A: A-273 | 15+375 | 1:25 | | | | | |
| B: trifluralin | 20+250 | 1:12.5 | | | | | |
| | 25+156.25 | 1:6.25 | | | | | |

**Table 40 Evaluation of indoor virulence and combined effect of compound A and acetochlor on digitaria sanguinalis (pre-emergence treatment)**

| Name of the agent | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 15+375 | 1:25 | 68.96 | 67.45 | 100.00 | 89.90 | 10.10 |
| B: acetochlor | 20+250 | 1:12.5 | 75.50 | 55.88 | 100.00 | 89.19 | 10.81 |
| A: A-273 | 15+375 | 1:25 | | | | | |
| B: acetochlor | 20+250 | 1:12.5 | | | | | |

**Table 41 Evaluation of indoor virulence and combined effect of compound A and fluchloraminopyrtefuryl on digitaria sanguinalis**

| Name of the agents | A+B dosage (g a.i./ha) | A: B | Fresh weight inhibition rate % | | | E0 | E-EO |
|---|---|---|---|---|---|---|---|
| | | | A single agent | B single agent | A+B, E | | |
| A: A-250 | 10+150 | 1:15 | | | | | |
| B:fluchloraminopyrtefuryl | 15+120 | 1:8 | | | | | |
| | 20+80 | 1:4 | | | | | |
| | 25+50 | 1:2 | | | | | |
| A: A-273 | 10+150 | 1:15 | 45.50 | 79.70 | 100.00 | 88.94 | 11.06 |
| B:fluchloraminopyrtefuryl | 15+120 | 1:8 | 59.30 | 74.76 | 100.00 | 89.73 | 10.27 |
| | 20+80 | 1:4 | 60.47 | 66.49 | 100.00 | 86.75 | 13.25 |
| | 25+50 | 1:2 | 62.48 | 47.40 | 100.00 | 80.26 | 19.74 |

**Table 42 Evaluation of indoor virulence and combined effect of compound A and fluroxypyr-meptyl on eclipta prostrata**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 2+80 | 1:40 | | | | | |
| | 4+60 | 1:15 | | | | | |
| B: fluroxypyr-meptyl | 8+40 | 1:5 | | | | | |
| | 16+32 | 1:2 | | | | | |
| A: A-273 | 2+80 | 1:40 | 28.12 | 73.18 | 100.00 | 80.72 | 19.28 |
| | 4+60 | 1:15 | 38.50 | 39.55 | 85.45 | 62.82 | 22.64 |
| B: fluroxypyr-meptyl | 8+40 | 1:5 | 46.05 | 34.55 | 100.00 | 64.69 | 35.31 |
| | 16+32 | 1:2 | 52.89 | 30.91 | 100.00 | 67.45 | 32.55 |

**Table 43 Evaluation of indoor virulence and combined effect of compound A and MCPA-sodium on eclipta prostrata**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | | | | | | | |
| B: MCPA-sodium | | | | | | | |
| A: A-273 | 4+160 | 1:40 | 38.50 | 45.00 | 86.82 | 66.17 | 20.65 |
| B: MCPA-sodium | 8+80 | 1:10 | 46.05 | 37.73 | 88.50 | 66.41 | 22.10 |

**Table 44 Evaluation of indoor virulence and synergistic effect of compound A and broclozone on echinochloa crusgalli**

| Name of the agents | A+B dosage (g a.i./ha) | A:B | Fresh weight inhibition rate % | | | E0 | E-EO |
|---|---|---|---|---|---|---|---|
| | | | A single agent | B single agent | A+B, E | | |
| A: A-250 | 10+150 | 1:15 | | | | | |
| | 15+120 | 1:8 | | | | | |
| B: broclozone | 20+80 | 1:4 | | | | | |
| | 25+50 | 1:2 | | | | | |
| A: A-273 | 10+150 | 1:15 | 19.41 | 44.95 | 68.07 | 55.64 | 12.44 |
| | 15+120 | 1:8 | 21.43 | 33.00 | 67.58 | 47.36 | 20.23 |
| B: broclozone | 20+80 | 1:4 | 30.04 | 16.30 | 84.25 | 41.44 | 42.81 |
| | 25+50 | 1:2 | 38.28 | 5.86 | 85.16 | 41.90 | 43.27 |

The bioassay results showed that E-E₀≥10 for Poaceae weeds, broadleaf weeds and cyperaceae weeds when such compounds were mixed with a variety of herbicides, which had obvious synergistic effects.

The above-mentioned examples are only preferred examples of the present invention. It should be pointed out that for one skilled in the art, some changes and improvements can be made without deviating from the idea of the present invention, which should be within the protection scope of the present invention.

## Claims

1. A herbicide composition of a uracil compound containing a carboxylate fragment, comprising an active ingredient A and an active ingredient B; wherein the active ingredient A is at least one compound indicated in formula (I), and the active ingredient B is at least one of a compound with a herbicidal activity or a salt thereof or a derivative thereof, different from the compound indicated in formula (I); a weight ratio of the active ingredient A to the active ingredient B is from 1:500 to 500:1; a structure of the active ingredient A is as follows: 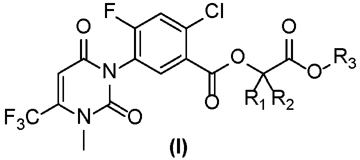 Wherein,
R₁ and R₂ are each independently selected from hydrogen or methyl; R₃ is selected from C_{1~6} alkyl, C_{1~6} halogen-substituted C_{1~6} alkyl, C_{3~6} alkenyl, C_{3~6} alkenyl substituted by one or more halogens, C_{3~6} alkynyl, C_{3~6} alkynyl substituted by one or more halogens, C_{3~6} cycloalkyl, C_{3~6} cycloalkyl C_{1~3} alkyl, C_{1~3} alkoxy C_{1~3} alkyl, C_{1~3} haloalkoxy C_{1~3} alkyl, C_{2~6} alkenoxy C_{1~3} alkyl, C_{2~6} haloalkenoxy C_{1~3} alkyl, C_{2~6} alkynoxy C_{1~3} alkyl, C_{2~6} haloalkynoxy C_{1~3} alkyl, C_{1~3} alkyl S(O)ₙ C_{1~3} alkyl, C_{3~6} oxygen containing cycloalkyl or C_{3~6} oxygen containing cycloalkyl C_{1~3} alkyl; n=0, 1 or 2; when R₁ is selected from hydrogen and R₂ is selected from methyl, the chiral carbon atom connected thereto is selected from either an R configuration or an S configuration, or a mixture of the two;
the active ingredient B is selected from quizalofop-P, haloxyfop-P, fluazifop-P, fenoxaprop-P, cyhalofop-butyl, quizalofop-P-tefuryl, quizalofop, haloxyfop-methyl, fenoxaprop, fluazifop-butyl, esprocarb, sethoxydim, cloproxydim, cycloxydim, clethodim, methoxyphenone, butroxydim, tepraloxydim, profoxydim, clodinafop-propargyl, pinoxaden, metamifop, propaquizafop, tralkoxydim, metsulfuron, chlorimuron, pyrazosulfuron, thifensulfuron, cinosulfuron, ethametsulfuron, cyclosulfamuron, ethoxysulfuron, rimsulfuron, primisulfuron, mesosulfuron-methyl, azimsulfuron, bensulfuron, chlorsulfuron, flazasulfuron, flucetosulfuron, foramsulfuron, halosulfuron, imazosulfuron, iodosulfuron-methyl-sodium, nicosulfuron, orthosulfamuron, oxasulfuron, prosulfuron, sulfosulfuron, triasulfuron, tribenuron, trifloxysulfuron, triflusulfuron, monosulfuron, iofensulfuron, tritosulfuron, propyrisulfuron, imazamethabenz, imazapic, imazapyr, imazaquin, flumetsulam, florasulam, metosulam, penoxsulam, pyroxsulam, bispyribac, pyribenzoxim, pyriftalid, pyriminobac-methyl, flucarbazone-sodium, propoxycarbazone-sodium, triafamone, imazethapyr, imazamox, KAI-177022, cloransulam-methyl, diclosulam, bispyribac-sodium, pyrithiobac-sodium, atrazine, prometryn, metribuzin, terbutryn, terbuthylazine, cyanazine, propazine, simazine, ametryn, simetryn, amicarbazone, isoproturon, chlorotoluro, diuron, daimuron, propanil, bentazone, bromoxynil octanoate, pyridate, bromacil, lenacil, terbacil, desmedipham, phenmedipham, bromoxynil, ioxynil, paraquat, diquat, saflufenacil, carfentrazone-ethyl, flumioxazin, fomesafen, fluoroglycofen, acifluorfen, oxyfluorfen, lactofen, oxadiazon, oxadiargyl, flumiclorac, sulfentrazone, pyraclonil, pentoxadiazon, butafenacil, fluthiacet-methyl, azafenidin, benzfendizone, bifenox, chlomethoxynil, cinidon-ethyl, fluazolate, flufenpyr-ethyl, flupoxam, KAI-162573(CAS NO.:2669821-71-8), SY-1604(CAS NO.:1949837-17-5), ethyl(5S)-3-(2-chloro-4-fluoro-5-(3-methyl-2,6-dioxo-4-(trifluoromethyl)-3,6-dihydropyrimidin-1(2H)-yl)phenyl)-5-methyl-4,5-dihydroisoxazole-5-carboxylate,ethyl(5R)-3-(2-chloro-4-fluoro-5-(3-methyl-2,6-dioxo-4-(trifluoromethyl)-3,6-dihydropyrimidin-1(2H)-yl)phenyl)-5-methyl-4,5-dihydroisoxazole-5-carboxylate, tiafenacil, trifludimoxazin, aclonifen, cyclopyranil, epyrifenacil, flufenoximacil, diflufenican, flurochloridone, flurtamone, norflurazon, fluridone, beflubutamid-M, mesotrione, tembotrione, benzobicyclon, tefuryltrione, lancotrione, sulcotrione, bicyclopyrone, topramezone, pyrasulfotole, benzofenap, pyrazolynate, pyrazoxyfen, tolpyralate, isoxaflutole, cypyrafluone, tripyrasulfone, flusulfinam, bipyrazone, fenpyrazone, fenquinotrione, benquitrione, Y13287(CAS NO.: 1639426-42-8), pyraquinate, clomazone, bixlozone, broclozone, beflubufamid, diflufenican, fluometuron, glyphosate, glufosinate-ammonium, glufosinate-P, bilanafos, asulam, butralin, trifluralin, dithiopyr, carbetamide, bethrodine, ethalfluralin, fluchloralin, oryzalin, pendimethalin, prodiamine, amiprophos, amiprofos-methyl, dimethyl tetrachloroterephthalate, propyzamide, carbetamide, chlorpropham, flamprop-methyl, flamprop-M-isopropyl, propham, butachlor, pretilachlor, mefenacet, anilofos, flufenacet, pyroxasulfone, s-metolachlor, acetochlor, piperophos, alachlor, amidochlor, ethachlor, thenylchlor, dimethenamid-P, metazachlor, metolachlor, pethoxamid, propachlor ESA sodium salt, propisochlor, mefenacet, diphenamid, naproanilide, devrinol, fentrazamide, cafenstrole, napropamide, fenoxasulfone, ipfencarbazone, indaziflam, methiozolin, napropamide-M, chlorthiamide, dichlobenil, flupoxan, indaziflam, isoxaben, triaziflam, dichlobenil, dinoterb, butylate, thiobencarb, vernolate, molinate, triallate, dimepiperate, asulam, cycloate, benfuresate, ethofumesate, 6-hydroxymelatonin, dalapon, EPTC, flupropanate, orbencarb, pebulate, 2,4-D, MCPA, 2,3,6-TBA, chloramben, dicamba, picloram, fluroxypyr, fluroxypyr-meptyl, quinclorac, quintrione, halauxifen-methyl, florpyrauxifen-benzyl, triclopyr, 3,6-dichloropicolinic acid, quinmerac, clacyfos, aminocyclopyrachlor, benazolin, chloramben-methyl, clomeprop, quinmerac, diflufenzopyr, fluchloraminopyr, cinmethylin, cyclopyrimorate, tetflupyrolimet, dimesulfazet, rimisoxafen or dioxopyritrione.

2. The herbicide composition according to claim 1, wherein the active ingredient B is selected from amicarbazone, paraquat, tribenuron, saflufenacil, topramezone, fenpyrazone, diflufenican, pyrazosulfuron, pyraquinate, bensulfuron, pretilachlor, oxadiargyl, flumioxazin, glufosinate-ammonium, glufosinate-P, bialaphos, benazolin, glyphosate, propanil, diquat, diuron, butachlor, pyroxsulam, oxadiazon, metamifop, pendimethalin, quinclorac, picloram, pyroxasulfone, flurtamone, tefuryltrione, bicyclopyrone, beflubutamid, flusulfinam, trifluralin, dithiopyr, halauxifen-methyl, triafamone, flucarbazone-sodium, butafenacil, haloxyfop-P-methyl, thiobencarb, cypyrafluone, cinmethylin, tembotrione, mesosulfuron-methyl, imazameth, imazamox, KAI-177022, fenoxaprop-P-ethyl, quizalofop-p-ethyl, s-metolachlor, benquitrione, chlorotoluron, halosulfuron, fluroxypyr, dicamba, imazethapyr, pyribenzoxim, bentazone, prometryn, metribuzin, cyhalofop-butyl, clodinafop-propargyl, thifensulfuron, tripyrasulfone, anilofos, bispyribac-sodium, florasulam, benzobicyclon, pyraclonil, bipyrazone, terbutylazine, terbutryn, penoxsulam, simetryne, clethodim, mesotrione, bromoxynil octanoate, nicosulfuron, acetochlor, fluoroglycofen, oxyfluorfen, isoproturon, clomazone, broclozone, isoxaflutole, atrazine, butralin, carfentrazone-ethyl, pinoxaden, 2,4-D, MCPA, bixlozone, cyclopyranil, fenisoxaflutole, lancotrione, pyrasulfotole, trifludimoxazin, sulfentrazone, Y13287(CAS NO.: 1639426-42-8), asulam, mefenacet, flufenacet, triaziflam, ethofumesate, florpyrauxifen-benzyl, flufenoximacil, fluchloraminopyr, epyrifenacil, KAI-162573(CAS NO.:2669821-71-8) or SY-1604(CAS NO.:1949837-17-5).

3. The herbicide composition according to claim 2, the active ingredient B is selected from glyphosate, glufosinate-ammonium, glufosinate-P, bialaphos, dicamba, bentazone, saflufenacil, paraquat, diquat, nicosulfuron, quizalofop-p-ethyl, metolachlor, acetochlor, metamifop, cyhalofop-butyl, atrazine, tribenuron, isoproturon, clomazone, broclozone, isoxaflutole, penoxsulam, sulfentrazone, Y13287(CAS NO.: 1639426-42-8), imazamox, KAI-177022, propanil, anilofos, mesotrione, topramezone, flusulfinam, fomesafen, butafenacil, trifluralin, pendimethalin, clodinafop-propargyl, pinoxaden, haloxyfop-P-methyl, clethodim, flumioxazin, oxadiargyl, fluoroglycofen, fluroxypyr, picloram, MCPA, 2,4-D, bispyribac-sodium, butachlor, epyrifenacil, KAI-162573(CAS NO.:2669821-71-8), SY-1604(CAS NO.:1949837-17-5), trifludimoxazin, fluchloraminopyr or flufenoximacil.

4. The herbicide composition according to claim 1, wherein, in a formula of the active ingredient A, R₁ and R₂ are selected from methyl; R₃ is selected from C_{1~6} alkyl, C_{3~6} alkenyl, C_{3~6} alkyne, C_{3~6} cycloalkyl, C_{3~6} cycloalkyl C_{1~3} alkyl, C_{1~3} alkoxy C_{1~3} alkyl, C_{2~6} alkenoxy C_{1~3} alkyl, C_{2~6} alkynoxy C_{1~3} alkyl, C_{1~3} alkyl S(O)ₙ C_{1~3} alkyl, C_{3~6} oxygen containing cycloalkyl or C_{3~6} oxygen containing cycloalkyl C_{1~3} alkyl; n=0, 1 or 2.

5. The herbicide composition according to claim 4, wherein, in the formula of the active ingredient A, R₁ and R₂ are selected from methyl; R₃ is selected from C_{1~6} alkyl, C_{1~3} alkoxy C_{1~3} alkyl, or C_{2~6} alkenoxy C_{1~3} alkyl.

6. The herbicide composition according to claim 1, wherein a weight ratio of the active ingredient A to the active ingredient B in the composition is from 1:300 to 48:1.

7. The herbicide composition according to claim 6, wherein the weight ratio of the active ingredient A to the active ingredient B in the composition is from 1:150 to 48:1.

8. The herbicide composition according to claim 1, wherein the weight percentage content of the active ingredient A and the active ingredient B in the composition is 1wt% to 95 wt% of the total.

9. The herbicide composition according to claim 8, wherein the weight percentage content of the active ingredient A and the active ingredient B in the composition is 10 wt% to 80 wt% of the total.

10. Use of the herbicide composition according to claim 1 in weed control.

11. The use according to claim 10, wherein the herbicide composition is applied to control weeds in useful crops selectively.

12. The use according to claim 11, wherein the useful crop is a genetically modified crop or a crop treated by genome editing technology.

13. The use according to claim 10, wherein the use is the use of the herbicide composition in controlling annual and perennial weeds, resistant or tolerant weeds in cultivated land, garden land or non-cultivated land.

14. A method of controlling an unwanted plant growth, comprising applying the herbicide composition of claim 1 to a plant, a plant part, a plant seed or a plant growth area.

15. The method according to claim 14, wherein the herbicide composition is used to control weeds in useful crops selectively.

16. The method according to claim 15, wherein the useful crop is a genetically modified crop or a crop treated by genome editing technology.
